# EUROPEAN PATENT APPLICATION

(11) **EP 1 982 679 A1**
(43) Date of publication of application: **22.10.2008**
(21) Application number: 07708062.0
(22) Date of filing: 05.02.2007
(51) Int. Cl.: A61F 13/49, A61F 13/15, A61F 13/494, A61F 13/511

(54) **ABSORPTIVE ARTICLE**

(30) Priority: 09.02.2006 JP 2006033116; 21.04.2006 JP 2006118554
(71) Applicant: JAPAN ABSORBENT TECHNOLOGY INSTITUTE, Chuo-ku, Tokyo 103-0007 (JP)
(72) Inventor: MORIYA, Reiko, Kawasaki-shi, Kanagawa 211-0062 (JP); SUZUKI, Migaku, Tokyo 103-0007 (JP)
(74) Representative: Grünecker, Kinkeldey, Stockmair & Schwanhäusser Anwaltssozietät
(86) International application number: PCT/JP2007/051944
(87) International publication number: WO 2007/091527

(57) **Abstract**

It is an object of the present invention to provide an absorbent article capable of separating urine and feces efficiently in use and having high urine absorption capacity. The object is attained by an absorbent article including: a first leak preventer in sheet form having a base part; a pair of side members extending in a longitudinal direction on right and left sides of the base part of the first leak preventer; an absorber comprised of at least one layer in an inner space formed by the base part of the first leak preventer and the side members, containing a super absorbent polymer, and capable of absorbing a body fluid; and a bridging member bonded to the pair of side members for bridging the side members, dividing the inner space into front and rear parts, capable of exhibiting a function of separating urine and feces, in which the bridging member is formed to be stretchable in a lateral direction.

## Description

### Technical Field

The present invention relates to a novel absorbent article.

### Background Art

An absorbent article such as a disposable diaper is an article for absorbing urine excreted from a wearer into an absorber employing an absorbing component such as wood pulp or a super absorbent polymer (hereinafter, may also be referred to as "SAP") and for receiving feces. A conventional absorbent article has no function of separating urine and feces, and thus urine and feces are liable to mix in use of the absorbent article. The mixing of urine and feces develops a problem of causing an offensive odor, a rash, or the like.

Correspondingly, various processes for separatingurine and feces have been proposed.

For example, there is proposed a process for separating urine and feces by a partition member (see Patent Documents 1 and 2, for example).

Further, there is proposed a process for separating urine from feces by: providing an opening in a rear part of an absorbent article; and receiving the feces in the opening (see Patent Documents 2 to 5, for example).

Further, there is proposed a process for inhibiting contact between urine and feces by absorbing the urine rapidly to remove the urine from an inner surface of an absorbent article (see Patent Documents 6 and 7, for example).
[Patent Document 1] JP 07-299092 A
[Patent Document 2] WO 02/24130
[Patent Document 3] JP 06-327715 A
[Patent Document 4] JP 06-343660 A
[Patent Document 5] JP 08-56986 A
[Patent Document 6] JP 06-90977 A
[Patent Document 7] JP 09-28732 A

### Disclosure of the Invention

However, the above-described conventional absorbent article is not capable of realizing efficient separation of urine and feces.

Therefore, it is an object of the present invention to provide an absorbent article capable of separating urine and feces efficiently in use.

### Means to Solve the Problems

The inventors of the present invention have conducted intensive studies for attaining the above-described object, and have completed an absorbent article having a novel structure.

That is, the present invention provides the following items (1) to (17).
(1) An absorbent article including:
   a first leak preventer in sheet form having a base part;
   a pair of side members extending in a longitudinal direction on right and left sides of the base part of the first leak preventer;
   an absorber comprised of at least one layer in an inner space formed by the base part of the first leak preventer and the side members, containing a super absorbent polymer, and capable of absorbing a body fluid; and
   a bridging member bonded to the pair of side members for bridging the side members, dividing the inner space into front and rear parts, capable of exhibiting a function of separating urine and feces, in which
   the bridging member is formed to be stretchable in a lateral direction.
(2) The absorbent article according to the above item (1), including a main body bonding member capable of bonding to the first leak preventer or the absorber at a lower end of the bridging member.
(3) An absorbent article including:
   a first leak preventer in sheet form having a base part;
   a pair of side members extending in a longitudinal direction on right and left sides of the base part of the first leak preventer;
   an absorber comprised of at least one layer in an inner space formed by the base part of the first leak preventer and the side members, containing a super absorbent polymer, and capable of absorbing a body fluid; and
   a bridging member bonded to the pair of side members for bridging the side members; and
   a urine/feces separating member dividing the inner space into front and rear parts, capable of exhibiting a function of separating urine and feces, in which
   the bridging member is formed to be stretchable in a lateral direction.
(4) An absorbent article including:
   a first leak preventer in sheet form having a base part;
   a pair of side members extending in a longitudinal direction on right and left sides of the base part of the first leak preventer;
   an absorber comprised of at least one layer in an inner space formed by the base part of the first leak preventer and the side members, containing a super absorbent polymer, and capable of absorbing a body fluid;
   a bridging member bonded to the pair of side members for bridging the side members; and
   a second leak preventer in sheet form present above the absorber on a rear side of the first leak preventer, in which
   the bridging member is formed to be stretchable in a lateral direction.
(5) The absorbent article according to the above item (4), including a feces stopping member in an upper surface or in a vicinity of a front end of the second leak preventer.
(6) The absorbent article according to the above item (4) or (5), including a main body bonding member bonding to the first leak preventer, the absorber, or the second leak preventer at a lower end of the bridging member.
(7) The absorbent article according to the above item (2) or (6), in which the bridging member and the main body bonding member are formed of a same material.
(8) The absorbent article according to any one of the above items (2), (6), and (7), in which the bridging member and/or the main body bonding member is stretchable.
(9) An absorbent article according to any one of the above items (1) to (8), in which the side members are formed of a pair of extended parts of the first leak preventer formed integrally with the base part and a pair of peripheral shape retaining members provided along peripheries of the extended parts.
(10) The absorbent article according to any one of the above items (1) to (9), in which the side members have stretchability.
(11) The absorbent article according to the above item (10), in which the side members are leg gathers provided on right and left ends of the base part.
(12) The absorbent article according to the above item (11), in which the leg gathers are standing leg gathers each including a leg part bonded to the base part and formed of a non-woven fabric, and a head part bonded to the leg part and including an elastic body.
(13) The absorbent article according to any one of the above items (1) to (12) , in which: the bridging member includes a stretchable area in a center part and non-stretchable areas on right and left sides of the stretchable area; and the bridging member is bonded to the side members in the non-stretchable areas.
(14) The absorbent article according to any one of the above items (1) to (12), in which: the side members are each stretchable; the bridging member is non-stretchable; and the side members are bonded to the bridging member so that the bridging member becomes stretchable in a lateral direction.
(15) The absorbent article according to any one of the above items (1) to (12), in which the side members and the bridging member are integrally formed of a stretchable material.
(16) The absorbent article according to the above item (15), in which the side members on right and left sides are bonded to each other in a longitudinal center part to form the bridging member.
(17) The absorbent article according to the above item (15) or (16), in which the side members and the bridging member form a front opening and a rear opening.

### Effect of the Invention

The absorbent article of the present invention is capable of separating urine and feces efficiently in use.

### Brief Description of the Drawings

[FIG. 1] A schematic developed plan view showing an absorbent article of the present invention according to an embodiment of a pull-on type diaper.
[FIG. 2] A lateral end view of the absorbent article of the present invention according to the embodiment of a pull-on type diaper taken along the X1-X1' line of FIG. 1.
[FIG. 3] A lateral end view of the absorbent article of the present invention according to the embodiment of a pull-on type diaper taken along the X2-X2' line of FIG. 1.
[FIG. 4] A lateral end view of the absorbent article of the present invention according to the embodiment of a pull-on type diaper taken along the X3-X3' line of FIG. 1.
[FIG. 5] A longitudinal end view of the absorbent article of the present invention according to the embodiment of a pull-on type diaper taken along the Y-Y' line of FIG. 1.
[FIG. 6] A schematic plan view showing a vicinity of a bridging member of the absorbent article of the present invention according to the embodiment of a pull-on type diaper of FIG. 1.
[FIG. 7] A schematic perspective view showing a situation that the absorbent article of the present invention according to the embodiment of a pull-on type diaper is worn.
[FIG. 8] A schematic developed plan view showing an absorbent article of the present invention according to another embodiment of a pull-on type diaper.
[FIG. 9] A lateral end view of the absorbent article of the present invention according to another embodiment of a pull-on type diaper taken along the X1-X1' line of FIG. 8.
[FIG. 10] A lateral end view of the absorbent article of the present invention according to another embodiment of a pull-on type diaper taken along the X2-X2' line of FIG. 8.
[FIG. 11] A lateral end view of the absorbent article of the present invention according to another embodiment of a pull-on type diaper taken along the X3-X3' line of FIG. 8.
[FIG. 12] A longitudinal end view of the absorbent article of the present invention according to another embodiment of a pull-on type diaper taken along the Y-Y' line of FIG. 8.
[FIG. 13] A schematic plan view showing a vicinity of a bridging member of the absorbent article of the present invention according to another embodiment of a pull-on type diaper of FIG. 8.
[FIG. 14] A schematic perspective view showing a situation that the absorbent article of the present invention according to another embodiment of a pull-on type diaper is worn.
[FIG. 15] A schematic developed plan view showing an absorbent article of the present invention according to an embodiment of a waist band-type diaper.
[FIG. 16] A lateral end view of the absorbent article of the present invention according to the embodiment of a waist band-type diaper taken along the X1-X1' line of FIG. 15.
[FIG. 17] A lateral end view of the absorbent article of the present invention according to the embodiment of a waist band-type diaper taken along the X2-X2' line of FIG. 15.
[FIG. 18] A lateral end view of the absorbent article of the present invention according to the embodiment of a waist band-type diaper taken along the X3-X3' line of FIG. 15.
[FIG. 19] A longitudinal end view of the absorbent article of the present invention according to the embodiment of a waist band-type diaper taken along the Y-Y' line of FIG. 15.
[FIG. 20] A schematic plan view showing a vicinity of a bridging member of the absorbent article of the present invention according to the embodiment of a waist band-type diaper of FIG. 15.
[FIG. 21] A schematic perspective view showing a situation that the absorbent article of the present invention according to the embodiment of a waist band-type diaper is worn.
[FIG. 22] Schematic plan views showing various examples of a bridging member having stretchable part in an entire lateral direction.
[FIG. 23] Schematic plan views showing various examples of a bridging member having a non-stretchable part in a lateral center part and having a stretchable part on right and left sides.
[FIG. 24] Schematic plan views showing various examples of a bridging member having a stretchable part in a lateral center part and having a non-stretchable part on right and left sides.
[FIG. 25] Schematic plan views showing various examples of a bridging member in which side members and the bridging member are integrally formed of a stretchable material.
[FIG. 26] Schematic longitudinal end views showing various examples of a shape of a bridging member.
[FIG. 27] Schematic longitudinal end views showing various arrangement examples of a main body bonding member.
[FIG. 28] Schematic lateral end views showing various arrangement examples of a main body bonding member.
[FIG. 29] Schematic longitudinal end views showing various shape and arrangement examples of a urine/feces separating member.
[FIG. 30] Schematic plan views showing various examples of a partly or entirely stretchable bridging member.
[FIG. 31] Schematic longitudinal end views showing various arrangement examples of a feces stopping member.
[FIG. 32] Schematic longitudinal end views showing various arrangement examples of a main body bonding member.
[FIG. 33] A schematic developed plan view showing an absorbent article of the present invention according to an embodiment of a waist band-type diaper.
[FIG. 34] A lateral end view of the absorbent article of the present invention according to the embodiment of a waist band-type diaper taken along the X-X' line of FIG. 33.
[FIG. 35] A schematic perspective view showing a situation that the absorbent article of the present invention according to the embodiment of a waist band-type diaper is worn.
[FIG. 36] A schematic developed plan view showing an absorbent article of the present invention according to an embodiment of a tape - type diaper. [FIG. 37] A lateral end view of the absorbent article of the present invention according to the embodiment of a tape-type diaper taken along the X-X' line of FIG. 36.
[FIG. 38] A schematic perspective view showing a situation that the absorbent article of the present invention according to the embodiment of a tape-type diaper is worn.
[FIG. 39] A schematic developed plan view showing an absorbent article of the present invention according to an embodiment of a pull-on type diaper.
[FIG. 40] A lateral end view of the absorbent article of the present invention according to the embodiment of a pull-on type diaper taken along the X-X' line of FIG. 39.
[FIG. 41] A schematic perspective view showing a situation that the absorbent article of the present invention according to the embodiment of a pull-on type diaper is worn.
[FIG. 42] Schematic diagrams each showing an arrangement of a base part of a first leak preventer and side members.
[FIG. 43] Schematic plan views each showing a positional relationship between side members and entirely stretchable bridging member.
[FIG. 44] Schematic plan views each showing a positional relationship between side members and entirely stretchable bridging member.
[FIG. 45] Schematic longitudinal end views showing various arrangement examples of a removable second leak preventer and/or feces disposal sheet.

### Legends

- 1: ABSORBENT ARTICLE
- 2: FIRST LEAK PREVENTER
- 3: ABSORBER
- 4: TOP SHEET
- 5: BRIDGING MEMBER
- 5', 25: SIDE MEMBERS
- 6: FECES STOPPING MEMBER
- 7: SECOND LEAK PREVENTER
- 8: STANDING LEG GATHER
- 9: WAIST GATHER
- 10: BONDING PART
- 11: LEG GATHER
- 12: SLIT
- 13: FRONT SEALING MEMBER
- 14: REAR SEALING MEMBER
- 15: HOOK MATERIAL
- 16: LOOP MATERIAL
- 17: GUIDE SHEET
- 18: OUTER LEG GATHER
- 19: PERIPHERAL SHAPE RETAINING MEMBER
- 20: WAIST BAND
- 21: MAIN BODY BONDING MEMBER
- 22: URINE/FECES SEPARATING MEMBER
- 23: BONDING TAPE
- 24: FECES DISPOSAL SHEET
- A: FIRST SKIN CONTACT SHEET
- B: SECOND SKIN CONTACT SHEET
- LH: LEG HOLE
- P: FRONT OPENING
- Q: REAR OPENING
- R: URINE RECEIVING PART
- S: FECES RECEIVING PART
- W: WAIST HOLE

### Best Mode for Carrying Out the Invention

Hereinafter, an absorbent article of the present invention will be described in more detail based on preferable embodiment modes shown in attached drawings. In the specification of the present invention, when the absorbent article is actually worn, a side close to a skin of a wearer is referred to as an "upper" side and a side far therefrom is referred to as a "lower" side. In addition, when the absorbent article is actually worn, a side corresponding to a front side of a body of a wearer is referred to as "front" and a side corresponding to a rear side thereof is referred to as "rear". In the drawings, members which are actually in contact with each other may be shown to be separately positioned for easy understanding.

An absorbent article according to the first aspect of the present invention basically includes: a first leakpreventer in sheet form having a base part; a pair of side members extending in a longitudinal direction on right and left sides of the base part of the first leak preventer; an absorber comprised of at least one layer in an inner space formed by the base part of the first leak preventer and the side members, containing a super absorbent polymer, and capable of absorbing a body fluid; and a bridging member bonded to the pair of side members for bridging the side members, dividing the inner space into front and rear parts, capable of exhibiting a function of separating urine and feces.

Materials generally used for a back sheet may be used as materials for the first leak preventer. Examples of the materials that can be used include: a resin film of PE, PP, PET, EVA, or the like; and a body fluid impermeable sheet such as a foamed sheet made of the resin. Further, a sheet having air permeability such as an air permeable film is preferably used as the body fluid impermeable sheet.

Further, the resin film may be used in a multilayer sheet of the film and a nonwoven fabric for the better touch or appearance. In this case, an SB nonwoven fabric, a thermal bond nonwoven fabric (for example, an air through type), or the like having a relatively light weight per unit area is preferably used as the nonwoven fabric.

Further, a multilayer sheet of the resin film and an absorber in sheet form described below may be used.

A highly water resistant non-woven fabric may also be used. Examples of the highly water resistant non-woven fabric include: SMS having a water resistance of 100 mmH₂O or more; and SMS having water resistance by filling fine gaps in amicro fiberwebwithmicrofibrillated cellulose (MFC) or wax. In this case, the non-woven fabric may be used alone, or used in a multilayer of a film and the highly water resistant non-woven fabric.

The first leak preventer may be formed of a plurality of members.

As described above, the first leak preventer has a base part. As described below, the first leak preventer has a pair of extended parts formed integrally with the base part of the first leak preventer, and the extended parts may form the side members.

In the absorbent article according to the first aspect of the present invention, the pair of side members extend in a longitudinal direction on right and left sides of the base part of the first leak preventer. The pair of side members are preferably bilaterally symmetric.

The pair of side members are preferably arranged such that respective ends are substantially parallel to each other. Thus, when the absorbent article is worn, the side members are positioned from a front side to a rear side along dents on both sides of a genital organ, groins, and to buttocks of a wearer so as to hold the wearer's crotch. Further, the bridging member may be contacted closely to a body surface of the wearer, and a distance between the side members (width of opening) may be maintained at a length required for receiving urine and feces.

When the ends of thepairof side members are substantiallyparallel to each other, easy production of the absorbent article is allowed.

As a preferred embodiment of the side members, the side members are formed of a pair of extended parts of the first leak preventer formed integrally with the base part, and a pair of peripheral shape retaining members provided along peripheries of the extended parts.

The pair of extended parts are not particularly limited as long as the extended parts stand upward from the right and left sides of the base part and are bent such that the respective peripheries come inside to form an inner space.

The peripheral shape retaining members may be provided along the entire length of peripheries of the extended parts. However, the positions of the peripheral shape retaining members are not particularly limited in the present invention, and the peripheral shape retaining members need not be provided in vicinities of front and rear ends of the peripheries of the extended parts.

The peripheral shape retaining members have a function of retaining peripheral shapes of the extended parts, to thereby contact the peripheries closely to a wearer' s body. In this way, leak of urine or feces out of the first leak preventer can be efficiently prevented. Further, when the absorbent article is worn, the peripheral shape retaining members can prevent the side members from becoming deformed and forming significant unevenness.

The shape of the peripheral shape retaining member is not particularly limited, but is preferably a narrow shape (such as a thread-like, belt-like, or tubular shape) having a width of 50 mm or less and preferably 2 to 25 mm, for example.

A material for the peripheral shape retaining member preferably has cushioning property (elasticity) and a certain thickness. Preferred examples thereof include: a foamed body (made of PU, PP, or PP/EVA, for example) ; a synthetic rubber sheet; a stretchable elastic film; a rubber filament; and a polyurethane filament. A gather may be formed with rubber thread, and the gather may be folded, to thereby obtain cushioning property and a certain thickness.

As another preferred embodiment of the side members, the side members having stretchability are given.

The side members having stretchability are not particularly limited, and examples thereof include leg gathers provided on right and left sides of the base part of the first leak preventer.

The leg gathers are not particularly limited, and conventionally known leg gathers can be used. Examples of the leg gathers include standing leg gathers each including: a leg part bonded to the base part and formed of a non-woven fabric; and a head part (head part having a thread-like or belt-like shape, for example) bonded to the leg part and including an elastic body. The non-woven fabric to be bonded to the base part preferably has water resistance, and more preferably has leak preventing property as well.

The inner space is formed by the base part of the first leak preventer and the side members on right and left sides.

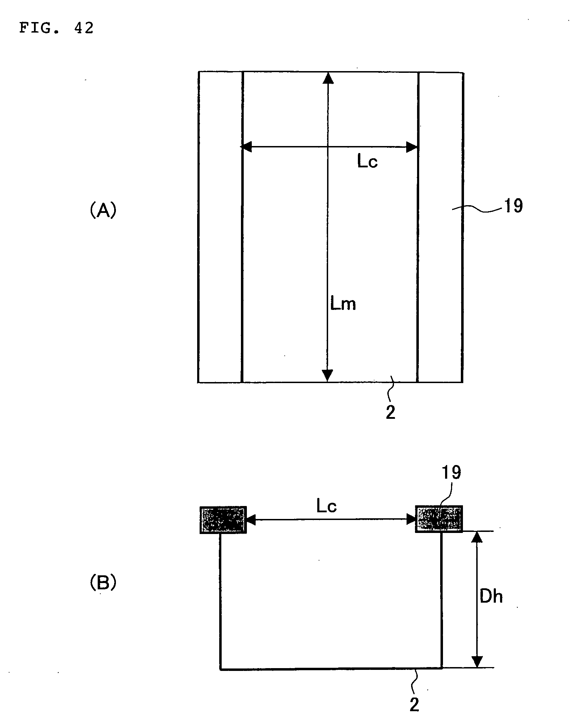

FIGs. 42 are schematic diagrams each showing an arrangement of the base part of the first leak preventer and the side members. FIG. 42 (A) is a plan view, and FIG. 42 (B) is a lateral end view. In FIGs. 42, the bridging member and the like are omitted.

In a first leak preventer 2 shown in FIGs. 42, a pair of extended parts are formed integrally with a base part. A pair of peripheral shape retaining members 19 are provided along the entire length of peripheries of the extended parts to form side members. The ends of the side parts are parallel to each other.

The first leak preventer 2 shown in FIGs. 42 has a narrow inner space in a longitudinal direction formed by the base part and the side members. Capability of the absorbent article to receive excretions such as urine and feces depends on a volume of the inner space.

A distance (Lc) between the side members and a length (Lm) of the side members in a longitudinal direction are determined depending on a size of a desired absorbent article and the like. A height (Dh) of the side members is determined depending on a thickness of an absorber to be used and the like.

The distance (Lc) between the side members is preferably long in view of easily receiving urine and feces. Specifically, in the case where the absorbent article is for an infant, Lc is preferably 25 mm or more, and more preferably 40 mm or more. Lc within the above ranges allows a urine receiving part on a front side to assuredly hold genital organs (penis and testes for boys, labia major for girls). Further, in a feces receiving part on a rear side, an upper part of the side members hardly gathers and intrudes into a dent formed by right and left buttocks to inhibit retaining of a shape of an opening.

The distance (Lc) between the side members is preferably 80 mm or less. Lc within the above range hardly forms a gap between the absorbent article and a wearer's body surface and causes no problems in contact. Further, a part of the buttocks to fall between the side members is small, and thus an area of the buttocks soiled by excretion of feces is small.

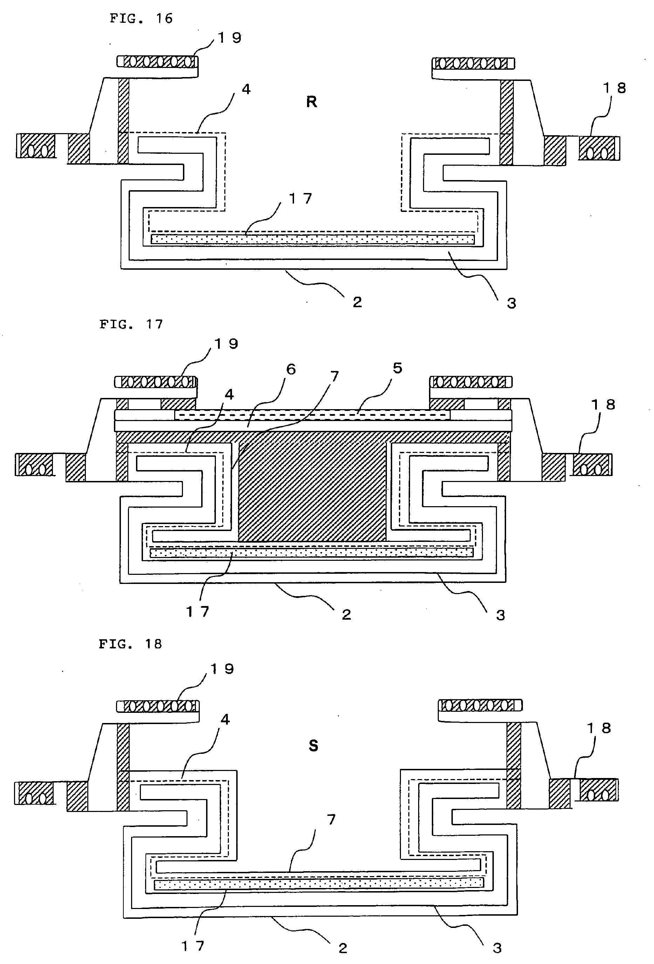

The side members may shift in a lateral direction or maybe deformed when the absorbent article is worn, and the bridging member bonded to and bridging the pair of side members is provided as described below for suppressing the degree of shift and deformation. As a preferred embodiment, an absorber in sheet form may be bonded to the extended parts of the first leak preventer and be integrated therewith, to thereby reinforce the side members and provide shape stability (see FIGs. 16 to 18).

The absorber is arranged in the inner space.

The absorber to be used in the present invention is not particularly limited as long as it contains a super absorbent polymer and is capable of absorbing a body fluid. For example, a powdery absorber formed of powderywoodpulp, unprocessed SAP, or the like maybe usedas the absorber, but the absorber is preferably an absorber in sheet form in consideration of shape stability, possibility of falling, and the like. Of those, a sheet absorber prepared by coating the super absorbent polymer on a non-woven fabric is preferred.

The type of absorber may appropriately be selected in accordance with applications. In the case where the absorbent article of the present invention is used as a baby diaper, for example, the absorbent article preferably has a large content of wood pulp for a newborn or younger infant (a size is referred to as S size) and preferably has a large SAP content for an older infant (a size is referred to as M size or L size).

As a preferred embodiment, the sheet absorber is preferably a super absorbent sheet containing 50 wt% or more SAP, preferably 60 to 95 wt% SAP.

The super absorbent sheet is an ultrathin sheet absorber containing SAP as a main component. The super absorbent sheet has a very high SAP content, and thus is very thin. The super absorbent sheet has a thickness of preferably 1.5 mm or less, more preferably 1 mm or less.

A structure or a production process for the super absorbent sheet is not particularly limited as long as the super absorbent sheet is an ultrathin sheet absorber containing SAP as a main component.

An example of the super absorbent sheet includes a super absorbent sheet obtained through an Air Laid process. The Air Laid process involves mixing pulverized wood pulp and SAP, adding a binder, and forming the mixture into a sheet, to thereby obtain a super absorbent sheet. Examples of the super absorbent sheet obtained through this process include: NOVATHIN (US trade mark) available from Rayonier Inc.; and B-SAP available from Oji Kinocloth Co., Ltd.

Another example of the super absorbent sheet includes a super absorbent sheet obtained through a process involving coating a body fluid permeable sheet such as a nonwoven fabric with SAP-dispersed slurry. The SAP-dispersed slurry is preferably prepared by dispersing SAP and microfibrillated cellulose (MFC) in a mixed solvent of water and ethanol. An example of the super absorbent sheet obtained through this process includes MegaThin (trade mark) available from Japan Absorbent Technology Institute.

Other examples of the super absorbent sheet include: a super absorbent sheet obtained through a process involving having a raised nonwoven fabric to carry a large amount of SAP and fixing the SAP with a hot melt binder, an emulsion binder, an aqueous fiber, or the like; a super absorbent sheet obtained through a process involving mixing fibrous SAP with a PET (polyethylene terephthalate) fiber and forming the mixture into a web; and an SAP sheet obtained by providing tissues above and below an SAP layer.

The absorber is provided in at least one layer. That is, the absorber may be comprised of one layer, or as two or more layers (multilayer).

Further, the absorber may be provided while being folded.

The absorbent article according to the first embodiment of the present invention is provided with a bridging member bonded to the pair of side members for bridging the side members and dividing the inner space into front and rear parts, capable of exhibiting a function of separating urine and feces. The bridging member also has an effect of maintaining the distance between the side members.

The bridging member is preferably partly or entirely stretchable.

A primary role of the bridging member is to bridge the side members on right and left sides for restricting lateral, vertical, and longitudinal motions of the side members to maintain a contact position between the side members (in the case where the side members include peripheral shape retaining members, the peripheral shape retaining members) and a wearer's body surface.

However, at the same time, the side members (in the case where the side members include peripheral shape retaining members, the peripheral shape retaining members) preferably have mobility corresponding to motions or changes in posture of a wearer. The bridging member is partly or entirely stretchable, to thereby provide such mobility.

A secondary role of the bridging member is to exhibit a sealing function allowing the absorbent article to constantly contact closely to a perineal region of a wearer for exhibiting the function of separating urine and feces, which is the object of the present invention. The bridging member is partly or entirely stretchable, to thereby maintain a contact even during motions or changes in posture of a wearer.

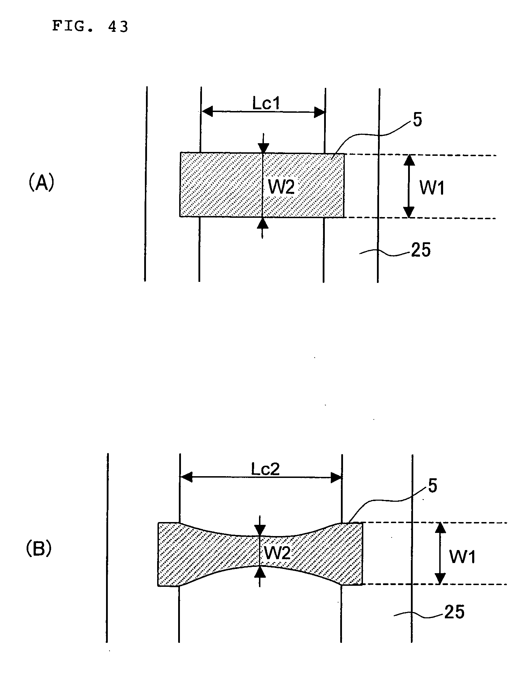

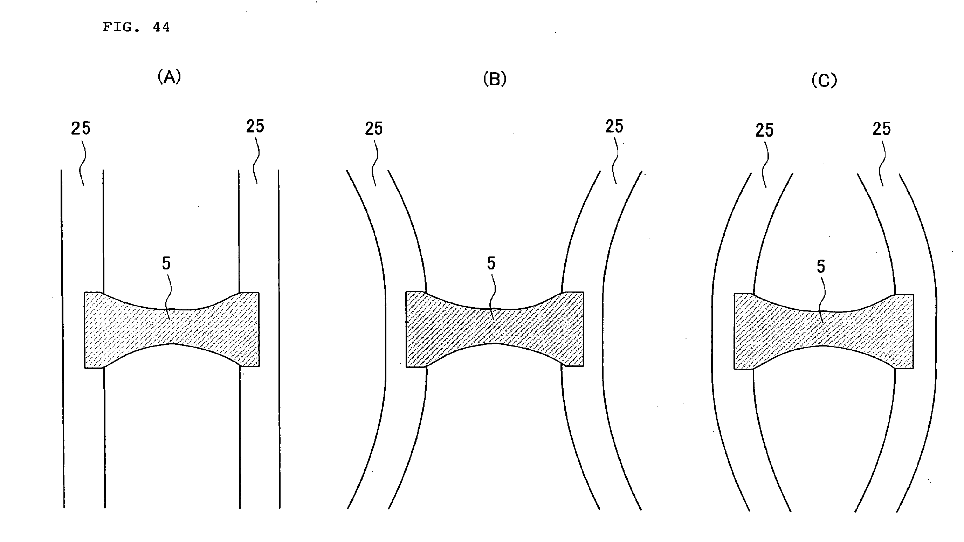

FIGs. 43 and 44 are schematic plan views each showing a positional relationship between side members and the bridging member which is entirely stretchable.

FIGs. 43 schematically show behaviors of the bridging member in a vicinity of a perineal region during shrinkage (FIG. 43(A)) and expansion (FIG. 43 (B)) in the case where the absorbent article is worn.

During shrinkage of the bridging member 5, a distance between side members 25 is Lc1, and a relationship between a width W1 of a bonding part between the bridging member 5 and the side members 25 and a width W2 of a lateral center part of the bridging member 5 is that W1 is nearly equal to W2 (see FIG. 43(A)).

During expansion of the bridging member 5, the distance between the side members 25 increases to Lc2 (that is, Lc2 > Lc1), and the relationship becomes W1 > W2.

The state and degree of deformation from shrinkage to expansion vary depending on design.

An expansion rate (Lc2/Lc1) of the distance between the side members 25 is preferably 120 to 300%, and more preferably 150 to 250%. An expansion rate within the above ranges easily exhibits a stretching effect. Further, the absorbent article does not excessively pressure the vicinity of a perineal region of a wearer during expansion, and a lump caused during shrinkage of the bridging member does not touch the vicinity of a perineal region the wearer, to thereby provide excellent wearability (comfortable fit).

The expansion rate (Lc2/Lc1) is arbitrarily set within the above ranges in consideration of the type, width, and thickness of the position of an elastic area, an elastic material to be used for the bridging member, and the like, and preferably in consideration of results of a doll test, a wearability test, and the like.

FIGs. 44 schematically show behaviors of the bridging member and the side members across the entire longitudinal length of the absorbent article during expansion of the bridging member.

The shapes of the side members vary depending on restricted states in front and rear parts of the absorbent article.

For example, in the case where positional restriction is little at a waist part of the absorbent article, the side members 25 are displaced while a substantially parallel state is maintained as shown in FIG. 44 (A).

In the case where the waist part of the absorbent article worn is expanded as in a waist band-type diaper, the side members 25 deform such that the distance therebetween increases toward front and rear directions as shown in FIG. 44(B).

In the case where the distance between the side members is fixed on front and rear ends as in a case where a pocket is formed at the waist part of the absorbent article, the side members 25 deform such that the distance therebetween at a center part alone increases and the distance therebetween decreases toward front and rear directions.

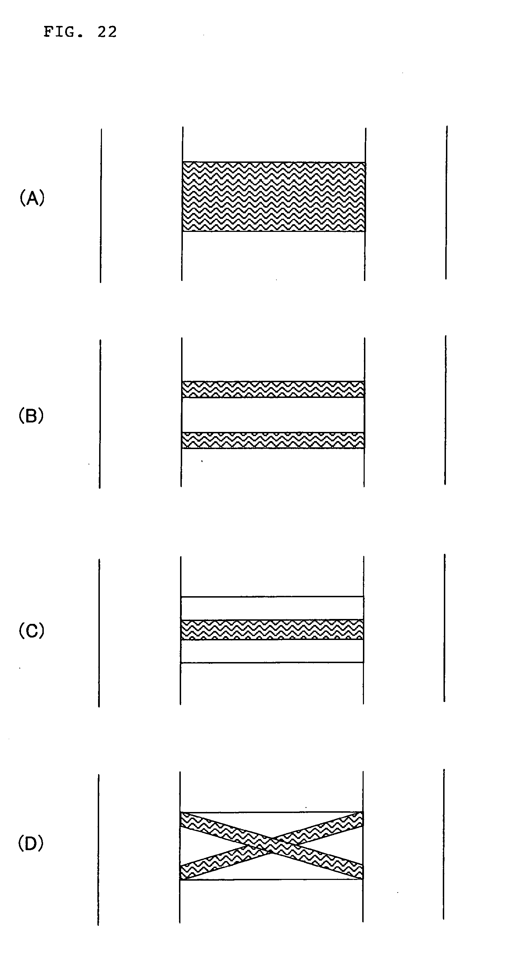

FIGs. 22 to 24 are schematic plan views showing various examples of the partly or entirely stretchable bridging member. Note that in the present invention, the term "non-stretchable part" includes a weakly stretchable part.

FIGs. 22 are schematic plan views showing various examples of a bridging member having stretchable part across an entire lateral direction. In FIGs. 22 (A) to 22 (D) , a wavy line represents a stretchable part formed of a stretchable material, and right and left areas represent the side members. The stretchable material is not particularly limited, but examples thereof include a urethane sheet and a urethane filament.

The bridging member shown in FIG. 22(A) is a stretchable part entirely formed of a stretchable material. The bridging member entirely stretches and contact along unevenness of a perineal region of a wearer.

The bridging member shown in FIG. 22 (B) includes a stretchable part on front and rear sides, and a non-stretchable part therebetween. The bridging member contacts along unevenness of the perineal region of the wearer, and the stretchable part on the front and rear sides has a high function of separating urine and feces.

The bridging member shown in FIG. 22 (C) includes anon-stretchable part on front and rear sides, and a stretchable part therebetween. In the bridging member, the stretchable part at a longitudinal center part fits into a dent of the perineal region of the wearer, and the non-stretchable part on the front and rear sides softly follow front and rear parts of the perineal region of the wearer.

The bridging member of FIG. 22(D) includes a stretchable part in an X-shaped part, and a non-stretchable part in a region excluding the X-shaped part. The bridging member contacts along unevenness of the perineal region of the wearer.

Note that FIGs. 22 (B) to 22 (D) are each a diagram showing a state where the bridging member is stretched in right and left directions and expanded. In the case where the bridging member is not stretched, the stretchable part shrinks and the non-stretchable part is wrinkled.

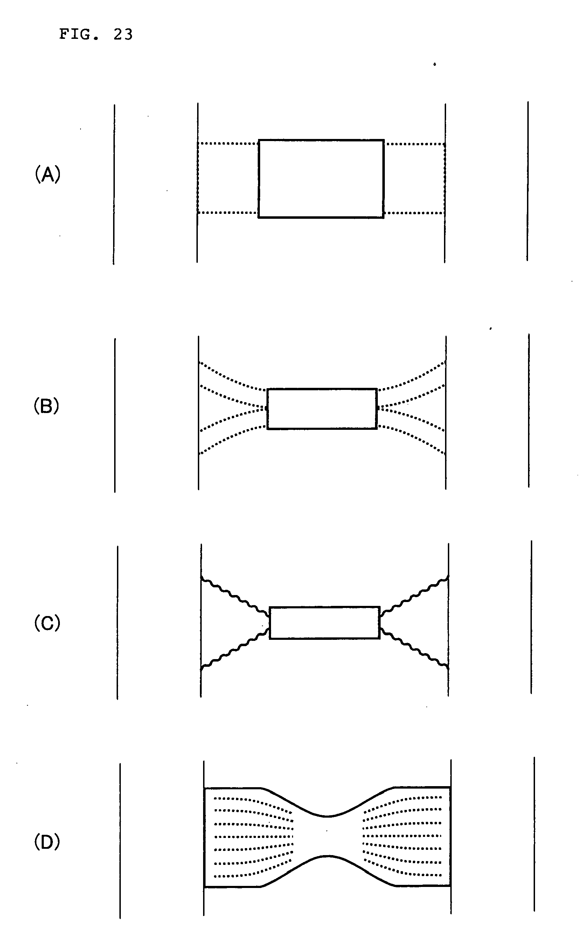

FIGs. 23 are schematic plan views showing various examples of a bridging member having a non-stretchable part in a lateral center part and having a stretchable part on right and left sides. In FIGs. 23(A) to 23(D), right and left areas represent the side members.

The bridging member shown in FIG. 23 (A) has a non-stretchable part in a lateral center part, and a stretchable part on right and left sides. The stretchable part is slightly shorter in a longitudinal direction than the non-stretchable part. A material for the non-stretchable part is not particularly limited, but an example thereof is a urethane foam having a certain thickness and cushioning property. A material for the stretchable part is not particularly limited, but an example thereof is a thin urethane film having stretchability.

The bridging member shown in FIG. 23(B) has a non-stretchable part in a lateral center part, and two wide stretchable parts extending in diagonal directions respectively on right and left sides (total of four parts). The material for the non-stretchable part is not particularly limited, but an example thereof is a PP foam. The material for the stretchable part is not particularly limited, but an example thereof is synthetic rubber.

The bridging member shown in FIG. 23 (C) has a non-stretchable part in a lateral center part, and two narrow stretchable parts extending in diagonal directions respectively on right and left sides (total of four parts). The material for the non-stretchable part is not particularly limited, but an example thereof is a PP non-woven fabric. The material for the stretchable part is not particularly limited, but an example thereof is rubber thread.

The bridging member shown in FIG. 23 (D) has a non-stretchable part in a lateral center part, and a stretchable part on right and left sides. The stretchable part is slightly longer in a longitudinal direction than the non-stretchable part. The bridging member can be obtained by arranging and bonding rubber thread on right and left sides alone of a non-woven fabric.

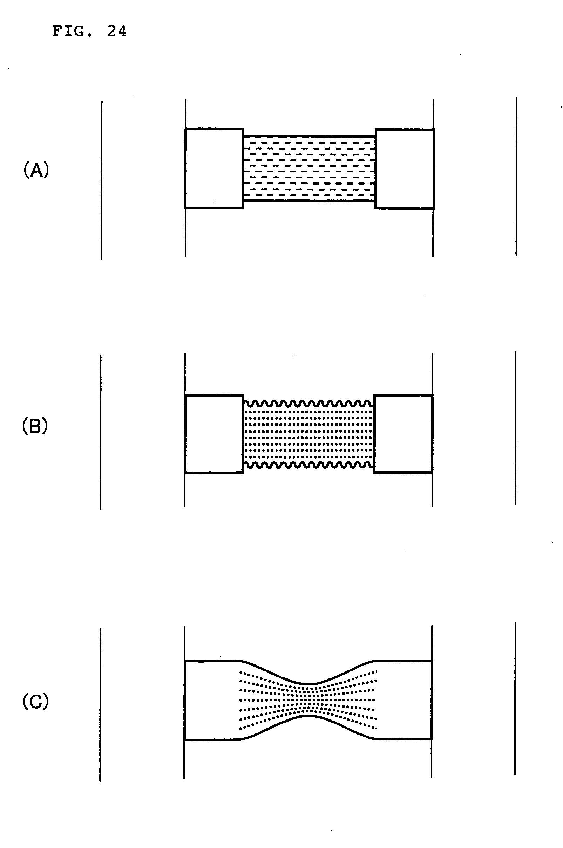

FIGs. 24 are schematic plan views showing various examples of a bridging member having a stretchable part in a lateral center part and having a non-stretchable part on right and left sides. In FIGs. 24(A) to 24(C), right and left areas represent the side members.

The bridging member shown in FIG. 24 (A) has a stretchable part in sheet form in a lateral center part, and a non-stretchable part on right and left sides. A material for the stretchable part is not particularly limited, but an example thereof is a synthetic rubber film.

The bridging member shown in FIG. 24 (B) has a wrinkled stretchable part in a lateral center part, and a non-stretchable part on right and left sides. For example, the stretchable part can be obtained by: arranging and bonding rubber thread as being expanded to a non-woven fabric; and relaxing to form wrinkles in the non-woven fabric.

The bridging member shown in FIG. 24(C) has a stretchable part in a lateral center part, and a non-stretchable part on right and left sides. The stretchable part is slightly shorter in a longitudinal direction than the non-stretchable part. The bridging member can be obtained by arranging and bonding a polyurethane filament only in a lateral center part of a non-woven fabric.

As a preferred embodiment of the bridging member, there is exemplified a bridging member having a stretchable area in a center part and a non-stretchable area on right and left sides of the stretchable area, and is bonded to the side members of the non-stretchable area. Examples thereof include the bridging members shown in FIGs. 24 (A) to 24 (C).

As another preferred embodiment of the bridging member, there is exemplified a bridging member including stretchable side members and non-stretchable bridging member, and is stretchable in a lateral direction by bonding stretchable side members and a non-stretchable bridging member.

As still another preferred embodiment of the bridging member, a bridging member includes the side members and the bridging member integrally formed of a stretchable material.

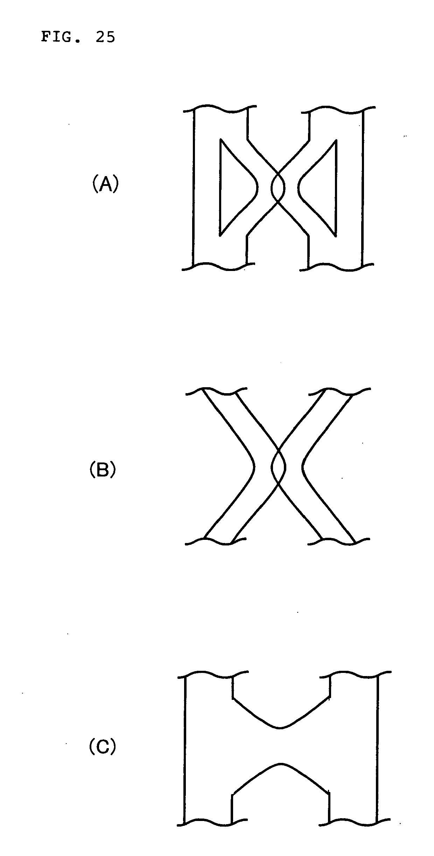

FIGs. 25 are schematic plan views showing various examples of a bridging member including the side members and the bridging member integrally formed of a stretchable material.

The side members and the bridging member integrally formed and shown in FIG. 25 (A) can be obtained by: providing a cut in a longitudinal direction in longitudinal center parts of the side members on right and left sides formed of a stretchable material; and pulling inner sides of the cut inward and bonding the inner sides together.

The side members and the bridging member integrally formed and shown in FIG. 25 (B) can be obtained by pulling the side members on right and left sides formed of a stretchable material inwardly in longitudinal center parts and bonding the side members together.

The side members and the bridging member integrally formed and shown in FIG. 25 (C) can be obtained by forming a bridging member formed of a stretchable material into a shape shown in the drawing.

In the absorbent article according to the first aspect of the present invention, the bridging member divides the inner space into front and rear parts and physically prevents transfer of urea and feces.

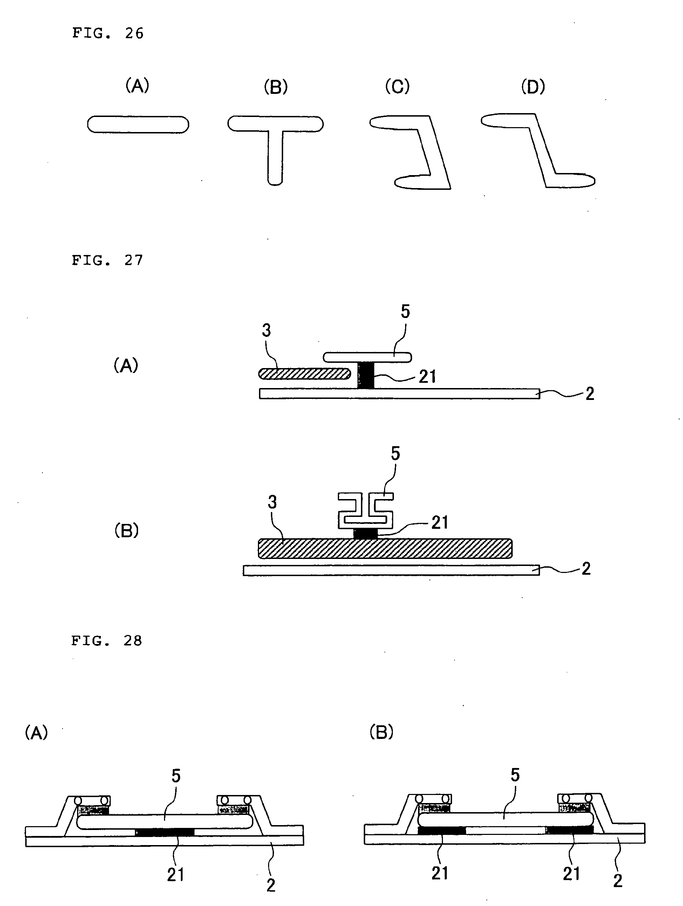

FIGs. 26 are schematic longitudinal end views showing various examples of a shape of the bridging member. In FIGs. 26, the left side corresponds to the front side of the absorbent article. Note that FIGs. 26 each show an end surface shape of a lateral center part of the bridging member, and a shape of the bridging member in a lateral direction needs not be identical thereto.

The bridging member shown in FIG. 26 (A) has a flat longitudinal end surface. For example, the back surface of the bridging member shown in FIG. 26(A) is bonded to the base part of the first leak preventer or the absorber, to thereby divide the inner space into front and rear parts and physically prevent transfer of urine and feces.

The bridging member shown in FIG. 26 (B) hasaT-shapedlongitudinal end surface. For example, the back surface of a part extending in a longitudinal direction of the bridging member shown in FIG. 26 (B) is bonded the base part of the first leak preventer or the absorber, to thereby divide the inner space into front and rear parts and physically prevent transfer of urine and feces.

The bridging member shown in FIG. 26 (C) has an inverted C-shaped longitudinal end surface. For example, the back surface of a lower part of the bridging member shown in FIG. 26 (C) is bonded to the base part of the first leak preventer or the absorber, to thereby divide the inner space into front and rearparts andphysicallyprevent transfer of urine and feces. Note that a slope of the member extending in a longitudinal direction in the drawing may be opposite to the slope shown in the drawing, or the member may be provided perpendicularly.

The bridging member shown inFIG. 26 (D) has a Z-shaped longitudinal end surface. For example, the back surface of a lower part of the bridging member shown in FIG. 26 (D) is bonded to the base part of the first leak preventer or the absorber, to thereby divide the inner space into front and rear parts and physically prevent transfer of urine and feces. Note that a slope of the member extending in a longitudinal direction in the drawing may be opposite to the slope shown in the drawing, or the member may be provided perpendicularly.

The bridging member may have at a lower end a main body bonding member for bonding to the first leak preventer or the absorber. Thus, separation of urine and feces can be conducted more efficiently.

A material for the main body bonding member is not particularly limited, and examples thereof include an adhesive, rubber, a film, foam (a foamed body), a non-woven fabric, and a net-like sheet.

In this case, as a preferred embodiment, the bridging member and the main body bonding material are formed of the same material. As another preferred embodiment, the bridging member and/or the main body bonding member is stretchable.

FIGs. 27 are schematic longitudinal end views showing various arrangement examples of the main body bonding member. In FIGs. 27, the left side corresponds to the front side of the absorbent article. Note that FIGs. 27 each show an end surface shape of a lateral center part of the absorbent article, and shapes of the respective members in a lateral direction need not be identical thereto.

In FIG. 27 (A) , the bridging member 5 is bonded to an upper surface of the first leak preventer 2 through a main body bonding member 21 and divides the inner space into front and rear parts. An absorber 3 is present on a front side of the divided inner space.

In FIG. 27 (B), the bridging member 5 is bonded to the absorber 3 present above the first leak preventer 2 through the main body bonding member 21 and divides the inner space into front and rear parts.

FIGs. 28 are schematic lateral end views showing various arrangement examples of the main body bonding member.

In FIG. 28 (A) , the bridging member 5 is bonded to an upper surface of the base part of the first leak preventer 2 through the main body bonding member 21 present in a lateral center part.

In FIG. 28 (B) , the bridging member 5 is bonded to an upper surface of the base part of the first leak preventer 2 through the main body bonding members 21 present on both right and left sides.

Note that in FIGs. 28 (A) and 28 (B) , an upper surface of the bridging member 5 is bonded on both right and left sides to inner surfaces of the side members of the first leak preventer 2.

In the absorbent article according to the first aspect of the present invention, the bridging member is formed to be stretchable in a lateral direction. Thus, the bridgingmember is capable of maintaining a state in contact with a perineal region of a wearer by following body motions of the wearer even during motion of the wearer such as opening and closing of legs. In this way, urine and feces can be separated efficiently.

The inventors of the present invention have conducted studies on a diaper for separatingurine and feces, and have acquired the following findings.

That is, a diaper for separating urine and feces to be used efficiently by various wearers must have a size appropriate for a wearer, taking into consideration differences between an adult and an infant, and a male and a female.

However, preparation of a diaper having a size appropriate for an individual wearer is commercially impossible, and efficient separation of urine and feces involves difficulties.

The size alone of the diaper cannot respond to the differences in body form of an individual wearer, changes in posture when the diaper is worn, and changes in motion of the wearer, and efficient separation of urine and feces involves difficulties.

The inventors of the present invention have found based on the findings described above that in a diaper for separating urine and feces, a bridging member for bridging side members arranged on right and left sides of a leak preventer and dividing an inner space of the diaper into front and rear parts, thus capable of exhibiting a function of separating urine and feces is provided as well as the bridging member is formed to be stretchable in a lateral direction, to thereby respond to the differences between an adult and a child and a male and a female, the differences in body form of an individual wearer, the changes in posture when the diaper is worn, and the changes in motion of the wearer. Thus, the inventors of the present invention have completed the absorbent article according to the first aspect of the present invention.

For the bridging member to exhibit the function of separating urine and feces, the bridging member must be maintained in a desired position, that is, in a state in contact with a perineal region of a wearer when the absorbent article is worn. A vicinity of the perineal region of the wearer is relatively uneven, and thus the bridging member must have following property allowing deformation in accordance with the unevenness.

The diaper is used invarious postures such as lying down, sitting, lying sideways, and lying on back, and in various motions such as crawling and walking. Thus, the bridging member must have following capability corresponding to various vertical, longitudinal, and lateral motions.

In the present invention, the bridging member is formed to be stretchable in a lateral direction, and the bridging member has excellent following capability. As a result, urine and feces can be separated efficiently.

Means for forming the bridging member to be stretchable in a lateral direction is not particularly limited. However, examples thereof include: means for forming the bridging member itself with a stretchable material; means for forming a member (such as side members, a first leak preventer, and a second leak preventer according to a third aspect of the present invention described below) which the bridging member is bonded to with a stretchable material; means for forming the bridging member to be stretchable in a lateral direction by using structures of members to which the bridging member is bonded and members in a vicinity thereof; and a combination thereof.

The absorbent article according to the second aspect of the present inventionbasically includes: a first leakpreventer in sheet form having a base part; a pair of side members extending in a longitudinal direction on right and left sides of the base part of the first leak preventer; an absorber comprised of at least one layer in an inner space formed by the base part of the first leak preventer and the side members, containing a super absorbent polymer, and capable of absorbing a body fluid; a bridging member bonded to the pair of side members for bridging the side members; and a urine/feces separating member dividing the inner space into front and rear parts, capable of exhibiting a function of separating urine and feces.

The absorbent article according to the second aspect of the present invention is basically similar to the absorbent article according to the first aspect of the present invention, and thus differences will only be described.

The absorbent article according to the second aspect of the present invention differs from the absorbent article according to the first aspect of the present invention in that the urine/feces separating member dividing the inner space into front and rear parts, capable of exhibiting a function of separating urine and feces is provided, and thus the function of separating urine and feces is not required for the bridging member. The absorbent article according to the second aspect of the present invention has the urine/feces separating member exhibiting a function of separating urine and feces, and thus urine and feces can be separated efficiently even if the bridging member has no function of separating urine and feces.

The urine/feces separating member is not particularly limited as long as it is capable of dividing the inner space into front and rear parts and physically preventing transfer of urine and feces. Examples thereof include an adhesive, rubber, a film, foam (a foamed body), a non-woven fabric, and a net-like sheet.

The inner space is separated into a urine receiving part and a feces receiving part by the urine/feces separating member, to thereby allow urine/feces separation treatment. Separation between the urine receiving part and the feces receiving part is preferably complete, but those parts may be connected if slightly or if an absorber inhibiting diffusion of urine is used, to thereby substantially allow urine and feces to be disposed separately.

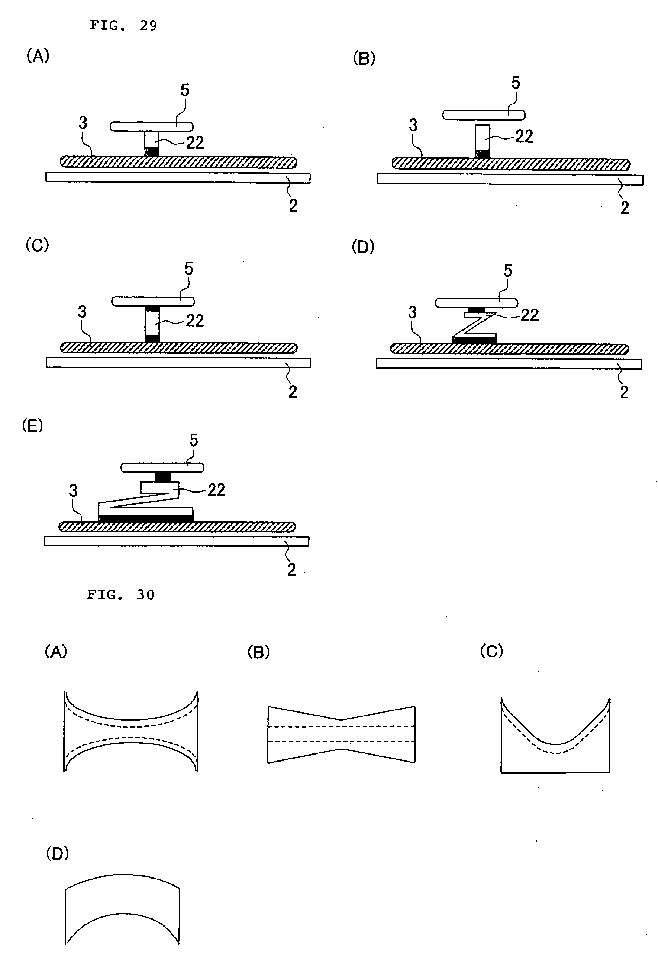

FIGs. 29 are schematic longitudinal end views showing various shape and arrangement examples of the urine/feces separating member. In FIGs. 29, the left side corresponds to the front side of the absorbent article. Note that FIGs. 29 each show an end surface shape of a lateral centerpart of the absorbent article, and shapes of the respective members in a lateral direction need not be identical thereto.

In FIG. 29 (A), a urine/feces separating member 22 in flat shape extending in a longitudinal direction is bonded to the back surface of the bridging member 5 and is bonded to an upper surface of the absorber 3 present above the first leak preventer 2 through an adhesive, to thereby divide the inner space into front and rear parts.

In FIG. 29 (B), the urine/feces separating member 22 in flat shape extending in a longitudinal direction is bonded to the upper surface of the absorber 3 present above the first leak preventer 2 through an adhesive, to thereby divide the inner space into front and rear parts. The urine/feces separating member 22 is not bonded to the back surface of the bridging member 5, and separation between the urine receiving part and the feces receiving part is not perfect. However, a gap between the urine/feces separating member 22 and the bridging member 5 is small, to thereby substantially allowurine and feces tobe disposed separately.

In FIG. 29 (C), the urine/feces separating member 22 in flat shape extending in a longitudinal direction is bonded to the back surface of the bridging member 5 through an adhesive and is bonded to the upper surface of the absorber 3 present above the first leakpreventer 2 through an adhesive, to thereby divide the inner space into front and rear parts.

In FIG. 29 (D), the urine/feces separating member 22 in Z-shape is bonded to the back surface of the bridging member 5 through an adhesive and is bonded to the upper surface of the absorber 3 present above the first leak preventer 2 through an adhesive, to thereby divide the inner space into front and rear parts.

In FIG. 29 (E), the urine/feces separating member 22 in Z-shape shape is bonded to the back surface of the bridging member 5 through an adhesive and is bonded to the upper surface of the absorber 3 present above the first leak preventer 2 through an adhesive, to thereby divide the inner space into front and rear parts. A lowerpart of the urine/feces separating member 22 in Z-shape shape largely projects forward. In this embodiment, in the case where feces is pushed forward by defecation in a sitting position or the like, the lower part of the urine/feces separating member 22 in Z-shape forms a bag to receive the feces.

The absorbent article according to the second aspect of the present invention may preferably employ the bridging member used for the absorbent article according to the first aspect of the present invention. However, other preferred bridging members will be described.

FIGs. 30 are schematic plan views showing various examples of a partly or entirely stretchable bridging member.

Bridging members shown in FIGs. 30 (A) to 30 (C) each have a structure in which a stretchable material (such as rubber) shown by a broken line is arranged inside a non-stretchable material (such as a non-woven fabric).

A bridging member shown in FIG. 30(D) is formed entirely of a stretchable material.

As shown in FIGs. 30 (A) to 30 (D), in the case where a longitudinal width of the bridging member is large on both right and left sides and small in a center part with a gradual change between the right and left sides and the center part, a supporting point at which the bridging member is in contact with a vicinity of a perineal region of a wearer is stabilized. Further, even in the case where a shape of the vicinity of a perineal region such as labia major of a female, in particular, a girl becomes narrower toward the her anus and the height difference from her groins becomes gradually smaller, the bridging member may be contacted closely to her body by utilizing an upper part of the labia major or roundness of the buttocks having a distinct height difference. In this case, if the longitudinal width of the bridging direction in a center part is set to be smaller than a distance between a urethral opening and anus, those effects are realized without blocking the urethral opening and the anus.

As shown in FIG. 30 (A), in the case where the stretchable material is arranged along front and rear ends of the bridging member, a front part contacts closely to roundness of the labia major or testes, and a rear part contacts closely to the buttocks.

As shown in FIG. 30 (B), in the case where the stretchable material is arranged in a longitudinal center part of the bridging member, the absorbent article is worn such that a stretchable part at the center is arranged at a dent of the perineal region. Thus, a front part is softly in contact with roundness of the labia major or the testes, and a rear part is softly in contact with the buttocks, to thereby reduce discomfort of the wearer.

As shown in FIG. 30 (C), a front end of the bridging member alone may have varying positions in a longitudinal direction.

As shown in FIG. 30 (D), front and rear ends of the bridging member may be curved (curved convex on a front side), to thereby utilize the thus-caused height difference.

The absorbent article according to a third aspect of the present invention basically includes: a first leakpreventer in sheet form having a base part; a pair of side members extending in a longitudinal direction on right and left sides of the base part of the first leak preventer; an absorber comprised of at least one layer in an inner space formed by the base part of the first leak preventer and the side members, containing a super absorbent polymer, and capable of absorbing a body fluid; a bridging member bonded to the pair of side members for bridging the side members; and a second leak preventer in sheet form present above the absorber on a rear side of the first leak preventer.

The absorbent article according to the third aspect of the present invention is basically similar to the absorbent article according to the first aspect of the present invention, and thus differences will only be described.

The absorbent article according to the third aspect of the present invention differs from the absorbent article according to the first aspect of the present invention in that a second leak preventer in sheet form present above the absorber on a rear side of the first leak preventer is provided, and thus the function of separating urine and feces is not required for the bridging member. The absorbent article according to the third aspect of the present invention is capable of receiving feces on the second leak preventer. Thus, urine and feces can be separated efficiently even if the bridging member has no function of separating urine and feces.

A material for the second leak preventer may employ the same material as that for the first leak preventer as described above. However, for stable disposal of feces in flux such as soft feces or watery feces containing a solid content dispersed in a fluid, a function of filtering the solid content by using a porous material is preferably provided. An example of a material having such a function is a composite sheet of a non-woven fabric and a fiber web (such as a composite sheet of SMS with a certain water resistance and a hydrophobic web) proposed in JP 2003-103677 A by the inventors of the present invention. Another example thereof is a polyethylene film with apertures having a funnel-shaped cross section inhibiting reverse flow, which is used as a top sheet of a sanitary napkin.

The second leak preventer may be formed of a plurality of members.

The second leak preventer may have a flat surface, for example, but its shape is not particularly limited. For example, the length and position in a longitudinal direction, a length in a lateral direction, and the like are not particularly limited. A specific preferred example of the shape is a container in which peripheries project upward. In this case, the feces is easily received on the second leak preventer.

As a preferred embodiment, the absorbent article according to the third aspect of the present invention may have a feces stopping member on an upper surface or in a vicinity of the front end of the second leak preventer. The feces stopping member may be bonded to or not bonded to the second leak preventer.

The feces stopping member efficiently prevents transfer of feces excreted into the feces receiving part to the urine receiving part.

A material for the feces stopping member is not particularly limited, and example thereof include an adhesive, rubber, a film, foam (a foamed body), a non-woven fabric, and a net-like sheet.

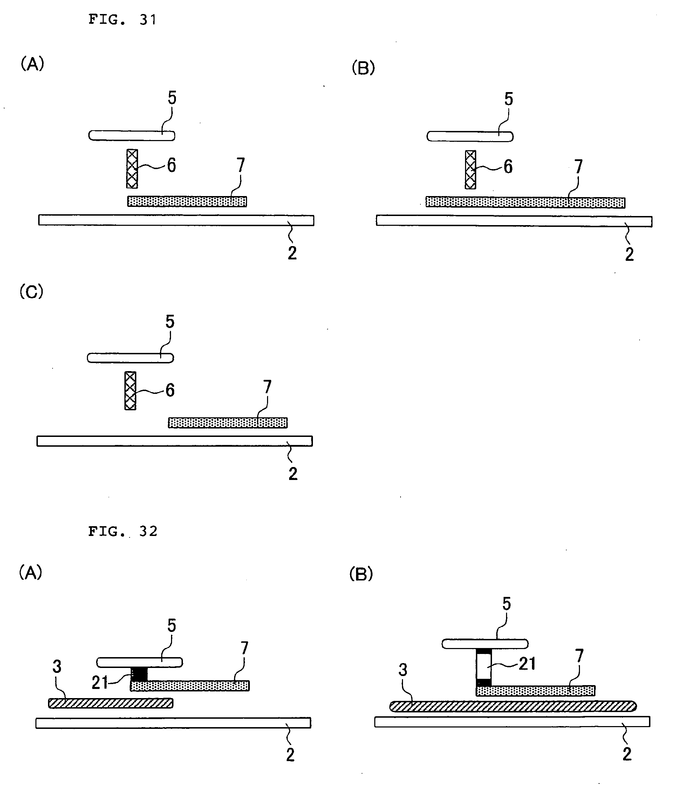

FIGs. 31 are schematic longitudinal end views showing various arrangement examples of the feces stopping member. In FIGs. 31, the left side corresponds to the front side of the absorbent article. Note that FIGs. 31 each show an end surface shape of a lateral center part of the absorbent article, and shapes of the respective members in a lateral direction need not be identical thereto.

In FIG. 31 (A), a feces stopping member 6 in flat shape extending in a longitudinal direction is present between the bridging member 5 and a front end of the second leak preventer 7 present above the first leak preventer 2.

In FIG. 31 (B), the feces stopping member 6 in flat shape extending in a longitudinal direction is present between the bridging member 5 and a front part of the second leak preventer 7 present above the first leak preventer 2.

In FIG. 31 (C), the feces stopping member 6 in flat shape extending in a longitudinal direction is present below the bridging member 5 and in front of the front end of the second leak preventer 7 present above the first leak preventer 2.

In FIGs. 31 (A) to 31 (C), the feces stopping member 6 is not bonded to the back surface of the bridging member 5 and is not bonded to the second leak preventer 7, so separation between the urine receiving part and the feces receiving part is not perferct. However, the gap between the feces stopping member 6 and the bridging member 5 or the second leak preventer 7 is small, to thereby substantially allow urine/feces separation treatment.

As a preferred embodiment, additionally, an absorber is arranged between the first leak preventer and the second leak preventer in the feces receiving part, and the absorber is provided continuous from the urine receiving part. In this way, a part of the feces receiving part present below the second leak preventer can also be used for absorption of urine, to thereby enhance urine absorbing ability.

In this case, if front and rear parts and/or a part of a side surface of the second leak preventer is provided with a passage communicating downward, when soft feces is received by the feces receiving part, the absorber present below the second leak preventer absorbs water of the soft feces through the passage, to thereby prevent leak of the soft feces.

In the absorbent article according to the third aspect of the present invention, the bridging member needs not have a function of separating urine and feces, but may have the function of separating urine and feces similarly to the bridging member used in the absorbent article according to the first aspect of the present invention.

The bridging member may have on its lower end a main body bonding member for bonding the first leak preventer, the absorber, or the second leak preventer.

In this case, as a preferred embodiment, the bridging member and the main body bonding to member are preferably formed of the same material. As another preferred embodiment, the bridging member and/or the main body bonding member is stretchable.

FIGs. 32 are schematic longitudinal end views showing various arrangement examples of the main body bonding member. In FIGs. 32, the left side corresponds to the front side of the absorbent article. Note that FIGs. 32 each show an end surface shape of a lateral center part of the absorbent article, and shapes of the respective members in a lateral direction need not be identical thereto.

In FIG. 32 (A), the bridging member 5 is bonded to an upper surface at a front end of the second leak preventer 7 through a bonding member 21 formed of an adhesive. The absorber 3 present above the first leak preventer 2 extends to a lower front part of the second leak preventer 7.

In FIG. 32 (B), the bridging member 5 is bonded to the upper surface at the front end of the second leak preventer 7 through the bonding member 21 and an adhesive provided above and below the bonding member 21. The absorber 3 present above the first leak preventer 2 extends beneath the second leak preventer 7 to the back side of the rear and thereof.

The second leak preventer may be removable. A feces disposal sheet may be provided on the second leak preventer, and the feces disposal sheet may be removable.

In general, a used diaper is disposed as garbage after excreted feces is flushed down the toilet or the like. In the present invention, the second leak preventer or the feces disposal sheet is removable, and thus excreted feces can efficiently be removed together with the second leak preventer or the feces disposal sheet from the absorbent article itself. In this case, the feces disposal sheet is preferably formed of a water-soluble sheet or a biodegradable material.

In the case where the second leak preventer and/or the feces disposal sheet is bonded to the bridging member, if there is employed as an embodiment a front part and a rear part of the bridging member are detachable, the second leak preventer and/or the feces disposal sheet and the rear part of the bridging member bonded thereto can be removed. In this case, the rear part of the bridging member also serves as a handle.

The front part and the rear part of the bridging member may be formed of the same material or formed of different materials. For example, the front part of the bridging member may have stretchability, and the rear part of the bridging member may have no stretchability. As a preferred embodiment, the rear part of the bridging member is formed of a water-soluble sheet or a biodegradable material.

The front part of the bridging member may serve as a urine stopping member. The rear part of the bridging member may serve as the feces stopping member.

The rear part of the bridging member may be bonded to one of the second leak preventer and the feces disposal sheet, or may be bonded to both.

The front part of the bridging member may be bonded only to the side members alone, or may also be bonded to the first leak preventer and/or the absorber.

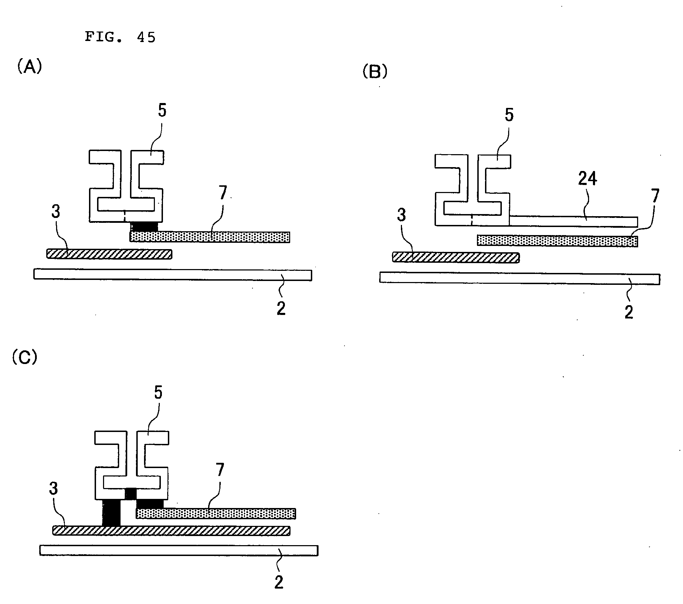

FIGs. 45 are schematic longitudinal end views showing various arrangement examples of a second leak preventer being removable and/or feces disposal sheet. In FIGs. 45, the left side corresponds to the front side of the absorbent article. Note that FIGs. 45 each show an end surface shape of a lateral center part of the absorbent article, and shapes of the respective members in a lateral direction need not be identical thereto.

In FIG. 45 (A), the bridging member 5 has an upside-down Ω-shaped longitudinal end, and a rear side of the back surface of the bridging member 5 is bonded to a front part of the second leak preventer 7. The bridging member 5 can be detached between the front part and the rear part (at a position of a dotted line in the drawing) along a cut line or the like. In the case where feces is excreted on the second leak preventer 7, the front part and the rear part are detached, and the second leak preventer 7 and the rear part of the bridging member may be removed and disposed.

In FIG. 45 (B), the bridging member 5 has an upside-down Ω-shape longitudinal end, and a rear part of the bridging member 5 is bonded to a front part of the feces disposal sheet 24 present above the second leak preventer 7. The bridging member 5 and the second leak preventer 7 are not bonded to each other. The bridging member 5 can be detached between the front part and the rear part (at a position of a dotted line in the drawing) along a cut line or the like. In the case where feces is excreted on the feces disposal sheet 24, the front part and the rear part are detached, and the feces disposal sheet 24 and the rear part of the bridging member may be removed and disposed.

In FIG. 45 (C), the bridging member 5 has an upside-down Ω-shaped longitudinal end, and a rear side of the back surface of the bridging member 5 is bonded to the front part of the second leak preventer 7. The front part and the rear part of the bridging member 5 are formed of different members and are weakly bonded to each other through an adhesive or the like. The bridging member 5 can be detached between the front part and the rear part. In the case where feces is excreted on the second leak preventer 7, the front part and the rear part are detached, and the second leak preventer 7 and the rear part of the bridging member may be removed and disposed.

The shape of the bridging agent having detachable front part and rear part is not limited to the upside-down Ω-shape as described above.

Hereinafter, the present invention will be described more specifically by referring to specific embodiments.

### <Embodiment 1>

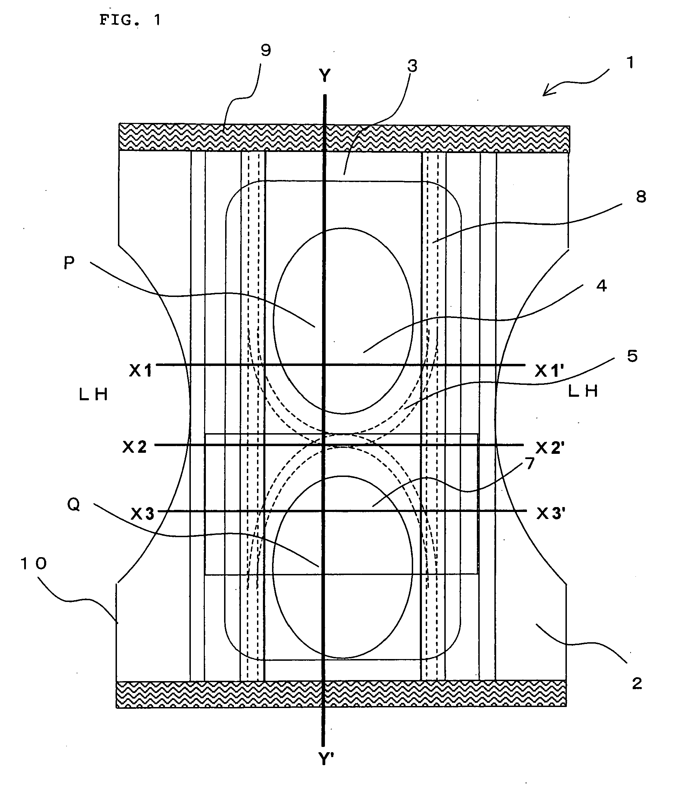

FIGs. 1 to 7 are schematic diagrams showing an absorbent article of the present invention according to an embodiment of a pull-on type diaper (tapeless-type diaper).

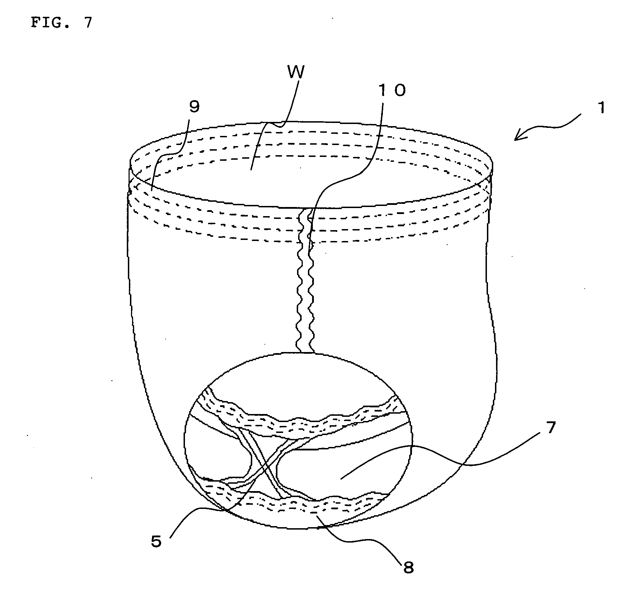

FIG. 1 is a developed plan view. Note that FIG. 1 is a developed view obtained by cutting a vicinity of a bonding part 10 shown in FIG. 7. In FIG. 1, a front side of the absorbent article is positioned on an upper side of the drawing.

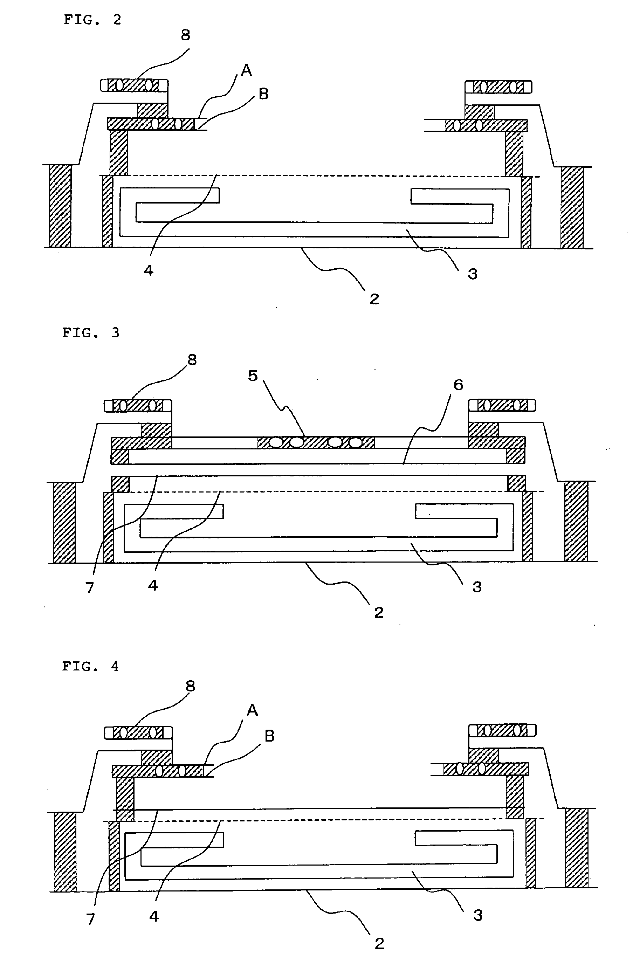

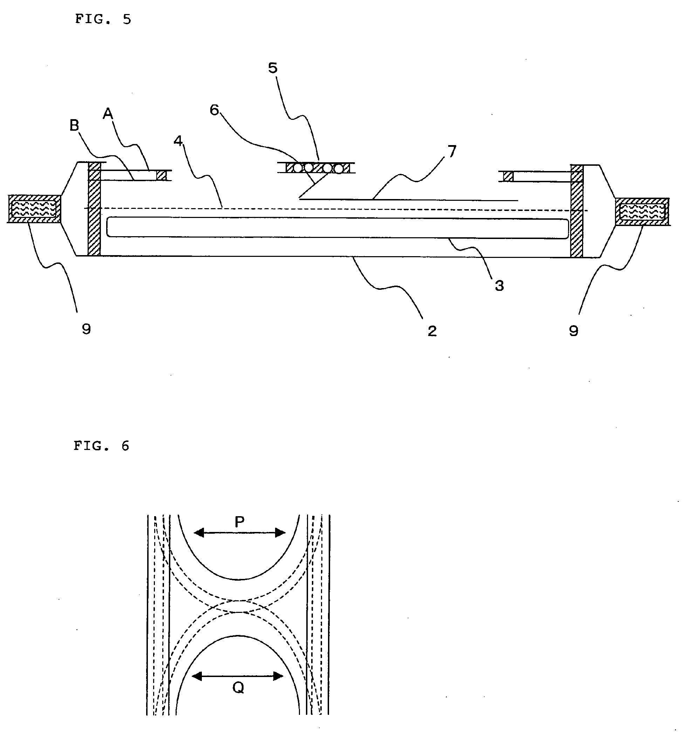

FIG. 2 is a lateral end view of the absorbent article taken along the X1-X1' line of FIG. 1, and FIG. 3 is a lateral end view of the absorbent article taken along the X2-X2' line of FIG. 1. FIG. 4 is a lateral end view of the absorbent article taken along the X3-X3' line of FIG. 1, and FIG. 5 is a longitudinal end view of the absorbent article taken along the Y-Y' line of FIG. 1.

FIG. 6 is a schematic plan view showing a vicinity of a bridging member 5 of the absorbent article of FIG. 1.

FIG. 7 is a perspective view showing a situation that the absorbent article is worn.

In FIGs. 1 to 7, a shaded part represents an adhesive.

The absorbent article 1 of the present invention basically includes: the first leak preventer 2 in sheet form having a base part; standing leg gathers 8 serving as a pair of side members extending in a longitudinal direction on right and left sides of the base part of the first leak preventer 2; the absorber 3 arranged in an inner space formed by the base part of the first leak preventer 2 and the standing leg gathers 8 serving as side members, containing a super absorbent polymer, and capable of absorbing a body fluid; and the bridging member 5 bonded to the standing leg gathers 8 serving as a pair of side members for bridging the standing leg gathers and dividing the inner space into front and rear parts, capable of exhibiting a function of separating urine and feces, in which the bridging member 5 and the standing leg gathers 8 have stretchability such that the bridging member 5 is stretchable in a lateral direction.

In the absorbent article 1 shown in FIG. 1, the absorber 3 in sheet form folded to be a C-shape is arranged on the first leak preventer 2 in sheet form having a base part and forming leg holes LH on right and left sides. Further, a top sheet 4 formed of liquid-permeable PP thermal bond (available from Rengo Co., Ltd., weight per unit area of 20 g/m², for example) is arranged thereon, and a skin contact sheet to be brought into contact with a wearer' s skin is arranged on an outermost surface thereof.

The skin contact sheet has a bilayer structure including a first skin contact sheet A as an upper layer and a second skin contact sheet B as a lower layer. The first skin contact sheet A and the second skin contact sheet B are both formed of hydrophobic PPSB (available from ToraySaehanInc., weight per unit of 13g/m², for example). A stretchable material described below is sandwiched between the first skin contact sheet A and the second skin contact sheet B.

On right and left sides of the base part of the first leak preventer 2, the standing leg gathers 8 serving as a pair of side members extend in a longitudinal direction.

The standing leg gathers 8 are not particularly limited, and conventionally known standing leg gathers may be used, for example. Specifically, the standing leg gathers are each formed of a side sheet of SMS (available from Avgol Ltd., weight per unit area of 13 g/m², for example) having two urethane filaments (available from DuPont-Toray Co., Ltd., 620 dtex, for example) attached at its end, for example. Right and left ends of the side sheets of the standing leg gathers 8 are bonded to the first leak preventer 2, and insides of the side sheets are bonded to an upper surface of the first skin contact sheet A such that standing and folded ends of the side sheets are arranged to be in contact with the wearer's skin.

In the first skin contact sheet A and the second skin contact sheet B, a front opening P to be used for passage of urine is provided in a front body part, and a rear opening Q to be used for passage of feces is provided in a back body part. Between the front opening P and the rear opening Q, the first skin contact sheet A, the second skin contact sheet B, and the bridging member 5 formedof a stretchable material and sandwiched by the skin contact sheets are formed and are bonded to the side sheets of the standing leg gathers 8 on right and left sides as described above for bridging the standing leg gathers.

When the absorbent article is worn, the bridging member 5 is positioned at a perineal region (between anus and genital organs) and separates urine and feces. For assured separation of urine and feces, a width of the bridging member 5 (length in a longitudinal direction) is 25 mm in a center part, and the width increases toward right and left sides.

Between the first skin contact sheet A and the second skin contact sheet B forming the bridging member 5, a stretchable material formed of two polyurethane filaments (available from DuPont-Toray Co., Ltd., 470 dtex, for example) each shown by chain linesis arranged in an X-shape from a rear left side of a periphery of the front opening P to a front right side of a periphery of the rear opening Q and from a rear right side of the periphery of the front opening P to a front left side of the periphery of the rear opening Q.

In each of the front body part and the back body part, the stretchable material is bonded to a lower side of a folded part of the standing leg gathers 8 through the first skin contact sheet A (see FIGs. 2 and 4).

In a center part, the stretchable material forms the bridging member 5 with the first skin contact sheet A and the second skin contact sheet B (see FIG. 3).

In the absorbent article 1, the bridging member 5 has a structure including a stretched material between the first skin contact sheet A and the second skin contact sheet B. Thus, the bridging member 5 itself is entirely stretchable. Further, the standing leg gathers 8 each have a structure including urethane filaments. Thus, the standing leg gathers 8 themselves are entirely stretchable.

Thus, the stretchability of the bridging member 5 itself and the stretchability of the standing leg gathers 8 to which the bridging member 5 is bonded allow the bridging member 5 to maintain a state in contact with a perineal region of the wearer following body motions of the wearer even during motion such as opening and closing of legs of the wearer. In this way, urine and feces can be separated efficiently.

In the absorbent article 1, the second leak preventer 7 in sheet form is arranged above the absorber 3 on a rear side of the first leak preventer 2 through the top sheet 4 (see FIG. 5).

The second leak preventer 7 is provided for receiving feces excreted from the wearer and preventing leak of the feces to a lower side in which the absorber 3 and others are present.

The second leak preventer 7 is formed of SMS (available from Avgol Ltd., weight per unit area of 18 g/m², water resistance of 120 mmH₂O, for example) and is arranged so as to substantially cover the entire rear opening Q from the lower side.

At a front end of the second leak preventer 7, the feces stopping member 6 bonded to the bridging member 5 is arranged. The feces stopping member 6 is formed of the same material as that of the second leakpreventer 7.

The feces stopping member 6 prevents the feces from transferring to a front side to leak out of the second leak preventer and mixing with urine, and prevents the urine received in the urine receiving part from transferring to a rear side to enter into the second leak preventer and mixing with the feces.

As shown in FIG. 7, in the absorbent article 1, the front body part and the back body part are bonded to each other at the bonding part 10, and the absorbent article 1 forms a pull-on type diaper provided with a waist gather 9 forming a waist hole W.

### <Embodiment 2>

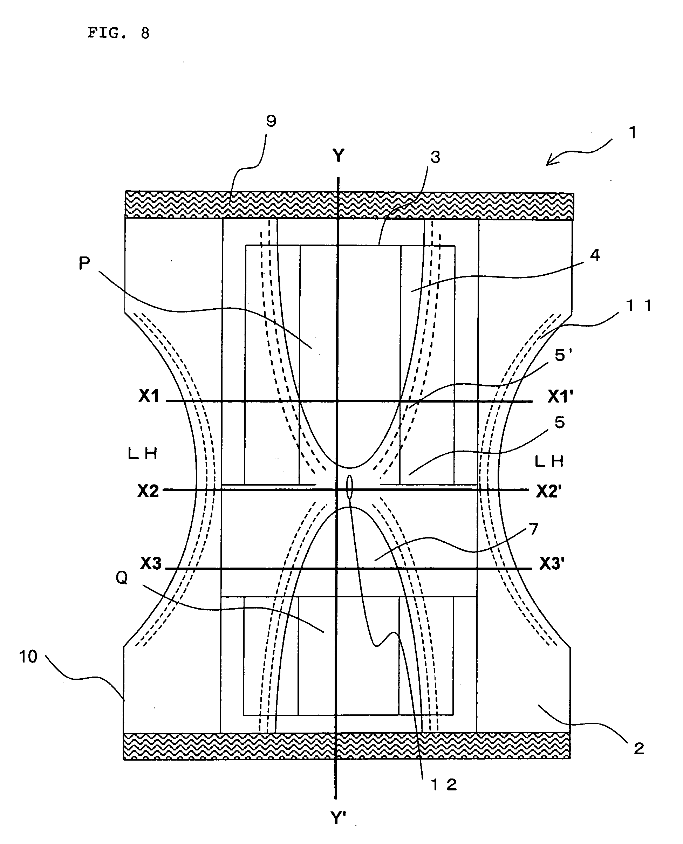

FIGs. 8 to 14 are schematic diagrams showing an absorbent article of the present invention according to another embodiment of a pull-on type diaper.

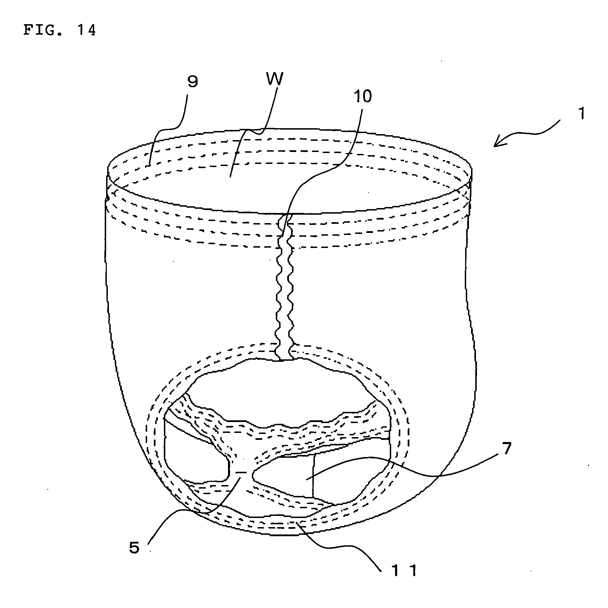

FIG. 8 is a developed plan view. Note that FIG. 8 is a developed view obtained by cutting a vicinity of the bonding part 10 shown in FIG. 14. In FIG. 8, a front side of the absorbent article is positioned on an upper side of the drawing.

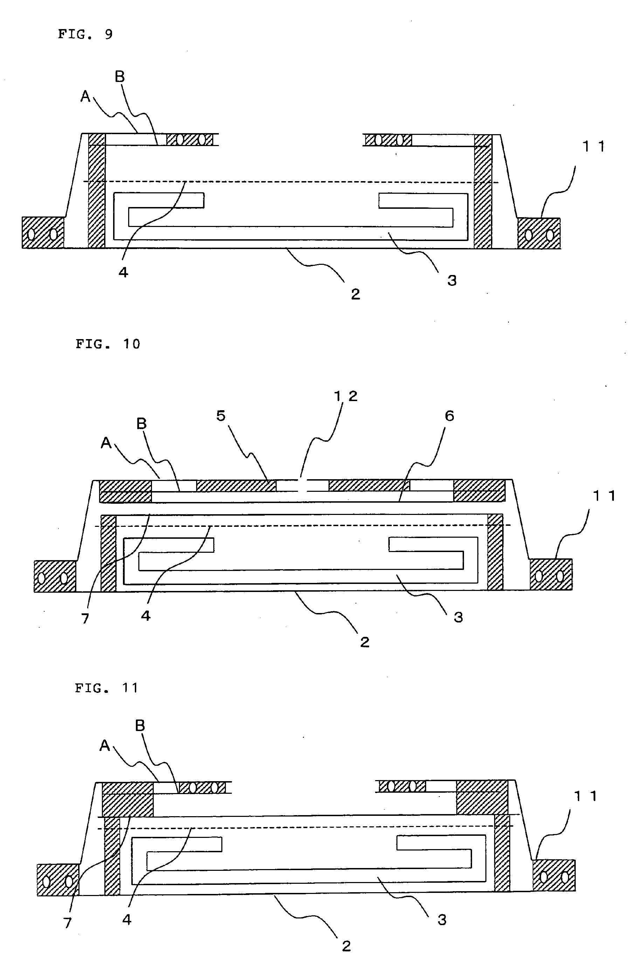

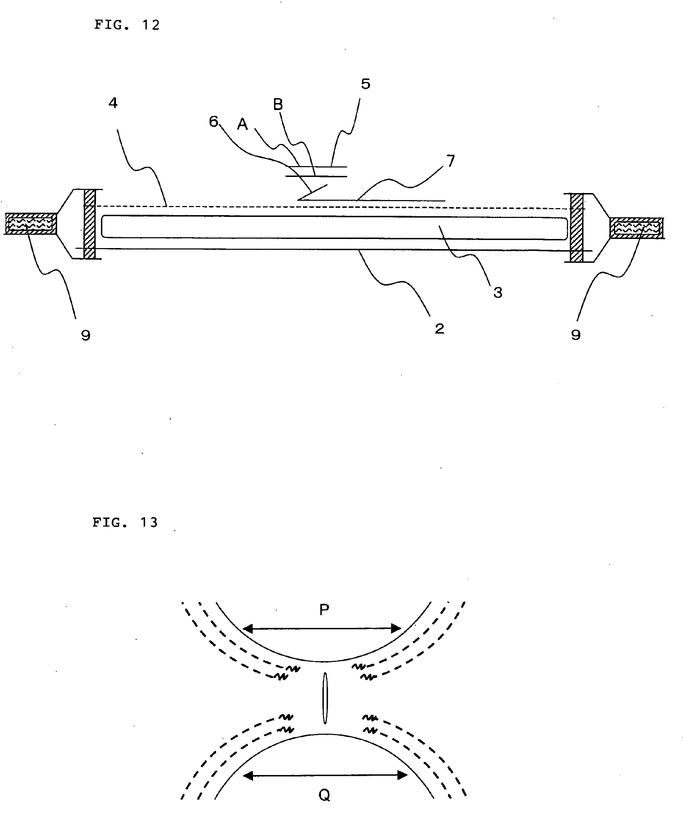

FIG. 9 is a lateral end view of the absorbent article taken along the X1-X1' line of FIG. 8, and FIG. 10 is a lateral end view of the absorbent article taken along the X2-X2' line of FIG. 8. FIG. 11 is a lateral end view of the absorbent article taken along the X3-X3' line of FIG. 8, and FIG. 12 is a longitudinal end view of the absorbent article taken along the Y-Y' line of FIG. 8.

FIG. 13 is a schematic plan view showing a vicinity of the bridging member 5 of the absorbent article of FIG. 8.

FIG. 14 is a perspective view showing a situation that the absorbent article is worn.

In FIGs. 8 to 14, a shaded part represents an adhesive.

The absorbent article 1 of the present invention basically includes: the first leak preventer 2 in sheet form having a base part; a pair of side members 5' extending in a longitudinal direction on right and left sides of the base part of the first leak preventer 2; the absorber 3 arranged in an inner space formed by the base part of the first leak preventer 2 and the side members 5', containing a super absorbent polymer, and capable of absorbing a body fluid; and the bridging member 5 bonded to the side members 5' for bridging integrally the side members and dividing the inner space into front and rear parts, capable of exhibiting a function of separating urine and feces, in which the bridging member 5 and the side members 5' have stretchability such that the bridging member 5 is stretchable in a lateral direction.

In the absorbent article 1 shown in FIG. 8, the absorber 3 in sheet form folded to be a C-shape is arranged on the first leak preventer 2 in sheet form having a base part and forming leg holes LH on right and left sides. Further, the top sheet 4 formed of liquid-permeable PP thermal bond (available from Rengo Co., Ltd., weight per unit area of 20 g/m², for example) is arranged thereon, and the skin contact sheet to be brought into contact with a wearer' s skin is arranged on an outermost surface thereof.

The skin contact sheet has a bilayer structure including a first skin contact sheet A as an upper layer and a second skin contact sheet B as a lower layer. The first skin contact sheet A is formed of hydrophobic PPSB (available from Toray Saehan Inc., mass per unit of 13 g/m², for example). The second skin contact sheet B is formed of water resistant PPSMS (available from Toray Saehan Inc., mass per unit of 15 g/m², for example). A stretchable material described below is sandwiched between the first skin contact sheet A and the second skin contact sheet B.

On right and left sides of the base part of the first leak preventer 2, leg gathers 11 extend in a longitudinal direction.

The leg gathers 11 are not particularly limited, and conventionally known leg gathers maybe used, for example . Specifically, the leg gathers are each formed by sandwiching two urethane filaments (available from DuPont-Toray Co., Ltd., 620 dtex, for example) between the first skin contact sheet A and the first leak preventer 2.

In the first skin contact sheet A and the second skin contact sheet B, a front opening P to be used for passage of urine is provided in a front body part, and a rear opening Q to be used for passage of feces is provided in a back body part. Between the front opening P and the rear opening Q, the first skin contact sheet A, the second skin contact sheet B, and a bridging member 5 formed of a stretched material and sandwiched by the skin contact sheets are formed. The stretchable material is not present in a lateral center part of the bridging member 5 and is present on right and left sides thereof.

The bridging member 5 is formed integrally with the side members 5' on right and left sides for bridging the side members.

When the absorbent article is worn, the bridging member 5 is positioned at a perineal region and separates urine and feces. For assured separation of urine and feces, a width of the bridging member 5 (length in a longitudinal direction) is 25 mm in a center part, and the width increases toward right and left sides.

Between the first skin contact sheet A and the second skin contact sheet B forming the bridging member 5, a stretchable material formed of two polyurethane filaments (available from DuPont-Toray Co., Ltd., 620 dtex, for example) each shown by chain lines is arranged along right and left peripheries of the front opening P and the rear opening Q. Two polyurethane filaments are arranged along the peripheries of the front opening P and the rear opening Q in a half circle. Next, the two polyurethane filaments are cut together with the first skin contact sheet A (also the second skin contact sheet B depending on a case) so as to provide a slit 12 in a longitudinal direction in a lateral center part so that the stretchable material is snapped back.

The stretchable material is arranged symmetrically in lateral along the peripheries of the front opening P and the rear opening Q of the first skin contact sheet A and the second skin contact sheet B in a front body part (see FIGs. 9 and 11), and is not present in the center part (see FIG. 10).

In the absorbent article 1, the bridging member 5 has a structure including a stretchable material between the first skin contact sheet A and the second skin contact sheet B, and no stretchable material in a lateral center part. Thus, the bridging member 5 itself is stretchable except in the center part. Further, the side members 5' formed integrally with the bridging member 5 have a structure including a stretchable material between the first skin contact sheet A and the second skin contact sheet B. Thus, the side members 5' themselves are entirely stretchable.

Thus, the stretchability of the bridging member 5 itself and the stretchability of the side members 5' to which the bridging member 5 is integrally bonded allow the bridging member 5 to maintain a state in contact with a perineal region of the wearer following body motions of the wearer even during motion such as opening and closing of legs of the wearer. In this way, urine and feces can be separated efficiently.

No stretchable material is present in a lateral center part of the bridging member. Thus, a part in contact with a urethral opening of a wearer is smooth and is easily contacted closely to the wearer's body. Further, SMS or the like having water resistance can be used for the second skin contact sheet B without hardening while flexibility of a non-woven fabric is maintained.

In the absorbent article 1, the second leak preventer 7 in sheet form is arranged above the absorber 3 on a rear side of the first leak preventer 2 through the top sheet 4 (see FIG. 12).

The second leak preventer 7 is provided for receiving feces excreted from the wearer and preventing leak of the feces to a lower side in which the absorber 3 and others are present.

The second leak preventer 7 is formed of SMS (available from Avgol Ltd., weight per unit area of 18 g/m², water resistance of 120 mmH₂O, for example) and is arranged so as to substantially cover the entire rear opening Q from the lower side.

The second leak preventer 7 is preferably formed of a polyethylene film with apertures having a funnel-shaped cross section and used as a top sheet of a sanitary napkin (available from Tredegar Corporation, 22Hex, for example).

At a front end of the second leak preventer 7, the feces stopping member 6 is bonded, and right and left ends of the feces stopping member 7 are bonded to the second skin contact sheet B. The feces stopping member 6 is formed of the same material as that of the second leakpreventer 7.

The feces stopping member 6 prevents the received feces from transferring to a front side to leak out of the second leak preventer and mixing with urine, and prevents the urine received in the urine receiving part from transferring to a rear side to enter into the second leak preventer and mixing with the feces.

As shown in FIG. 14, in the absorbent article 1, the front body part and the back body part are bonded to each other at the bonding part 10, and the absorbent article 1 forms a pull-on type diaper provided with the waist gather 9 forming the waist hole W.

### <Embodiment 3>

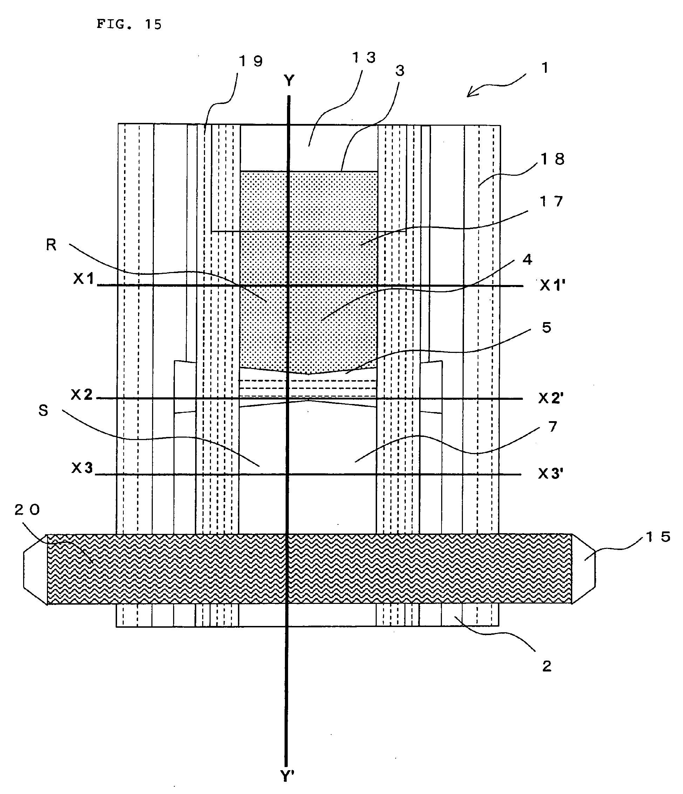

FIGs. 15 to 21 are schematic diagrams showing an absorbent article of the present invention according to an embodiment of a waist band-type diaper.

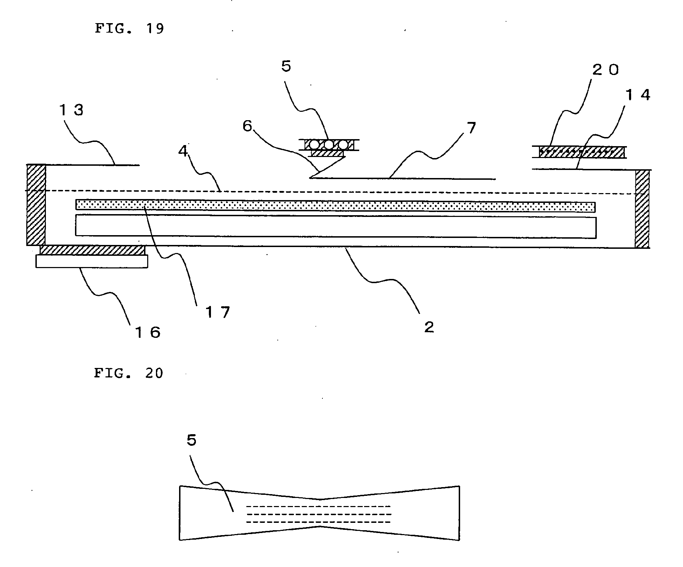

FIG. 15 is a developed plan view. Note that in FIG. 15, a front side of the absorbent article is positioned on an upper side of the drawing. FIG. 16 is a lateral end view of the absorbent article taken along the X1-X1' line of FIG. 15, and FIG. 17 is a lateral end view of the absorbent article taken along the X2-X2' line of FIG. 15. FIG. 18 is a lateral end view of the absorbent article taken along the X3-X3' line of FIG. 15, and FIG. 19 is a longitudinal end view of the absorbent article taken along the Y-Y' line of FIG. 15.

FIG. 20 is a schematic plan view showing a vicinity of a bridging member 5 of the absorbent article of FIG. 15.

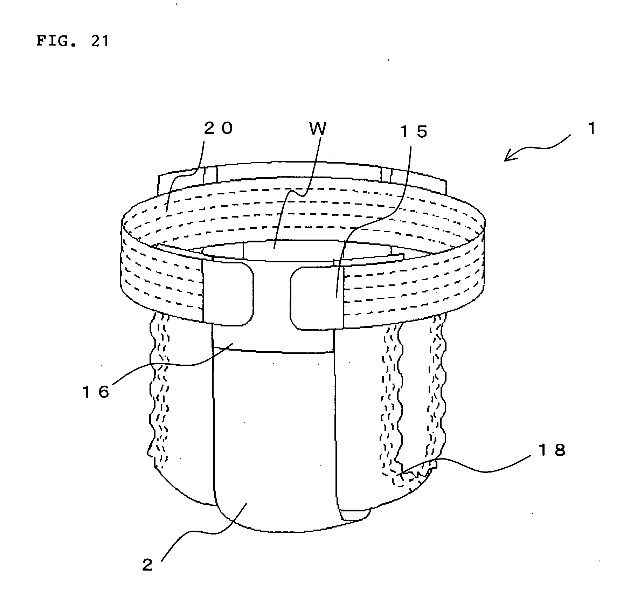

FIG. 21 is a perspective view showing a situation that the absorbent article is worn.

In FIGs. 15 to 21, a shaded part represents an adhesive.

The absorbent article 1 shown in FIG. 15 basically includes: the first leak preventer 2 in sheet form having a base part; a pair of side members formed of outer leg gathers 18 and peripheral shape retaining members 19 extending in a longitudinal direction on right and left sides of the base part of the first leak preventer 2; the absorber 3 arranged in an inner space formed by the base part of the first leak preventer 2 and the side members formed of the outer leg gathers 18 and the peripheral shape retaining members 19, containing a super absorbent polymer, and capable of absorbing a body fluid; and the bridging member 5 bonded to the peripheral shape retaining members 19 forming the pair of side members for bridging the peripheral shape retaining members and dividing the inner space into front and rear parts, capable of exhibiting a function of separating urine and feces, in which the bridging member 5 and the peripheral shape retaining members 19 have stretchability such that the bridgingmember 5 is stretchable ina lateral direction.

In the absorbent article 1 shown in FIG. 15, the absorber 3 in sheet form is arranged on the first leak preventer 2 in sheet form having a base part, and the top sheet 4 formed of liquid-permeable PP thermal (available from Rengo Co., Ltd., weight per unit area of 20 g/m², for example) is arranged thereon. The absorber 3 in sheet form and the top sheet 4 are each folded to be a Z-shape on right and left sides, and the top sheet 4 is bonded to an extended part of the first leak preventer to be integrated for reinforcement of the side members (see FIGs. 16 to 18). Further, in a lateral center part, a guide sheet 17 is arranged between the absorber 3 and the top sheet 4.

The guide sheet 17 has a passage for transferring urine.

In use of an absorbent article, an amount of urine to be absorbed by an absorber may significantly be uneven by position depending on posture of a wearer, a worn position, and the like. In such a case, if the absorbent article of the present invention includes a guide sheet, urine transfers from a part of an absorber containing a large amount of urine to a part containing a small amount thereof. As a result, unevenness is reduced. Thus, substantial urine absorption amount and usable time of the absorbent article can be increased.

The guide sheet 17 is not particularly limited as long as it has a structure including a passage allowing transfer of urine. However, the guide sheet 17 preferably has no body fluid absorbing property or body fluid retaining property for rapid transfer of urine. A preferred example thereof is a concavity-and-convexity-containing sheet member (apertures-provided concavity-and-convexity-containing sheet member) having apertures at convex portions.

The apertures-provided concavity-and-convexity-containing sheet member has at least one uneven surface having concave portions and convex portions. In the apertures-provided concavity-and-convexity-containing sheet member, many continuous concave portions serve as flow paths of a body fluid. The apertures-provided concavity-and-convexity-containing sheet member has an advantage in that even when some convex portions are deformed in use, transfer of a body fluid is not inhibited.

Further, in the apertures-provided concavity-and-convexity-containing sheet member, apertures also serve as flow paths.

An example of a material for the apertures-provided concavity-and-convexity-containing sheet member includes a body fluid impermeable film composed of a resin such as PE, PP, PVA, or urethane.

An example of the apertures-provided concavity-and-convexity-containing sheet member includes one suggested by the inventors of the present invention in WO 02/065965.

When an apertures-provided concavity-and-convexity-containing sheet member is used as a guide sheet, a body fluid may remain in apertures causing a liquid residue, that is, a rewetting value may become high. In this case, a hydrophilic diffusion sheet is preferably provided beneath the apertures-provided concavity-and-convexity-containing sheet member in combination therewith. Thus, a body fluid present in the apertures permeates and transfers through the hydrophilic diffusion sheet, to thereby drastically decrease a rewetting value. Further, the apertures-provided concavity-and-convexity-containing sheet member is prevented from shifting from the leak preventer, and its form stability improves as well.

A hydrophilic nonwoven fabric product having a relatively low basis weight is preferably used as the hydrophilic diffusion sheet. Examples of the hydrophilic nonwoven fabric product include: a hydrophilic nonwoven fabric containing rayon, cotton, wood pulp, or the like; and tissue paper. Examples thereof include: tissue paper; a rayon spun bond (TCF, available from Futamura Chemical Co., Ltd., for example) ; a cotton spun lace, a rayon/ PP mixed spun lace ; and rayon/ PET mixed spun lace.

Further examples thereof include a synthetic nonwoven fabric such as a spun bond nonwoven fabric of PP, PE, or PET; a thermal bond nonwoven fabric of PP; and a through-air nonwoven fabric of PE/PET subjected to hydrophilic treatment through surface treatment with a surfactant or the like.

The apertures-provided concavity-and-convexity-containing sheet member and the hydrophilic diffusion sheet maybe laminated through simple superposition or may be combined through hot melting, heat laminating, or the like.

A body fluid impermeable sheet is preferably provided beneath the hydrophilic diffusion sheet in combination therewith. Thus, the body fluid is not even temporarily store between the hydrophilic diffusion sheet and the sheet of the leak preventer and is quickly transferred to a bag of the leak preventer. An example of a material for the body fluid impermeable sheet includes a film made of a resin such as PE, PP, PVA, or urethane.

The hydrophilic diffusion sheet and the body fluid impermeable sheet may be laminated through simple superposition or may be combined through hot melting, heat laminating, or the like.

Further, the apertures-provided concavity-and-convexity-containing sheet member and the body fluid impermeable sheet may be used in combination therewith, instead of using the hydrophilic diffusion sheet.

The apertures-provided concavity-and-convexity-containing sheet member and the body fluid impermeable sheet maybe laminated through simple superposition or may be combined through hot melting, heat laminating, or the like.

On right and left sides of the base part of the first leak preventer 2, the outer leg gathers 18 and the peripheral shape retaining members 19 forming the pair of side members extend in a longitudinal direction.

The outer leg gathers 18 are not particularly limited, and conventionally known leg gathers may be used, for example. Specifically, the outer leg gathers are each formed of a side sheet of SMS (available from Avgol Ltd., weight per unit area of 13 g/m², for example) having two urethane filaments (available fromDuPont-TorayCo. , Ltd. , 620 dtex, for example) attached at its ends, for example. Right and left ends of the side sheets of the outer leg gathers 18 are bonded to the first leak preventer 2, and insides of the side sheets are bonded to an upper surface of the bridging member 5, and further, the side sheets are arranged such that the peripheral shape retaining members 19 rising and folded back is brought in contact with the wearer's skin.

The peripheral shape retaining members 19 are wide and formed by folding a pair of right and left peripheries inside the side sheet outward to wrap five urethane filaments (available from DuPont-Toray Co., Ltd., 470 dtex, for example).

In the present invention, the peripheries of side members preferably follow body motions of the wearer. Thus, the peripheral shape retaining members preferably employ one or both of the following two embodiments.

The first embodiment refers to peripheral shape retaining members formed by folding peripheries of the side members outward. In this embodiment, a fold serves as a fulcrum so that a folded part tend to move loosely, and the peripheral shape retaining members have excellent flexibility.

The second embodiment refers to peripheral shape retaining members themselves having stretchability. The peripheral shape retaining members themselves may have stretchability by using a stretchable member such as rubber thread, flat state rubber (such as natural rubber), or a stretchable member in sheet form and arbitrarily selecting the thickness, material, and the like of the stretchable member. The peripheral shape retaining members having stretchability are not particularly limited in size, shape, and the like. As a preferred embodiment, the peripheral shape retaining members each preferably have a band-like shape. In general, a width of each of the band-like peripheral shape retaining members having a band-like shape is preferably 2 to 80 mm, more preferably 5 to 40 mm for an infant and 20 to 60 mm for an adult.

As a preferred embodiment of the peripheral shape retaining members described above, the peripheral shape retaining members 19 is formed into bands having stretchability and the bands are arranged parallel on right and left (employing both embodiments described above), for example. In this way, the peripheral shape retaining members 19 contact closely to dents on both sides of testes of a male or contact closely to dents on both sides of labia major of a female, and thus the bridging member having stretchability can be positioned along roundness of the testes or the labia major without a gap.

The peripheral shape retaining members 19 are each maintained to be stretched in a lateral direction by a waist band or a side tape shown in FIG. 15 at a front end and a rear end. Thus, even if the wearer has legs closed, a distance between the right and left peripheral shape retaining members 19 (width of opening) may be maintained at a length required for receiving urine and feces.

The inner space formed by the base part of the first leak preventer 2, and the outer leg gathers 18 and the peripheral shape retaining members 19 forming the side members is a channel-like shape from a front body part to a back body part. The inner space is divided into front and rear parts by the bridging member 5. The front part serves as a urine receiving part R, and the rear part serves as a feces receiving part S.

When the absorbent article is worn, the bridging member 5 is positioned at a perineal region and separates urine and feces. For assured separation of urine and feces, a width of the bridging member 5 (length in a longitudinal direction) is 20 mm in a center part, and the width increases toward right and left sides (see FIG. 20).

The bridging member 5 has a structure in which a stretchable material formedof three urethane filaments (available from DuPont-Toray Co., Ltd., 470 dtex, for example) is arranged in a lateral center part between two SMS's (available from Avgol Ltd., weight per unit area of 13 g/m², for example).

In the absorbent article 1, the second leak preventer 7 in sheet form is arranged above the absorber 3 at a rear side of the first leak preventer 2 through the top sheet 4 (see FIG. 15).

The second leak preventer 7 is provided for receiving feces excreted from the wearer and preventing leak of the feces to a lower side in which the absorber 3 and the like are present.

The second leak preventer 7 is formed of PPSB (available from Toray Saehan Inc., mass per unit of 13 g/m², for example) having a PE film laminated thereon, and is arranged across the entire feces receiving part S.

At a front end of the second leak preventer 7, the feces stopping member 6 to be bonded to the bridging member 5 is arranged. The feces stopping member 6 is formed of the same material as that of the second leak preventer 7.

The second leak preventer 7 preferably employs a composite sheet obtained by entangling and laminating a PET web (such as a web of 6D/51 mm staple fiber, weight per unit area of 20 g/m²) with SMS (available from Avgol Ltd. , weight per unit area of 15 g/m², water resistance of 120 mmH₂O, for example). In this case, the second leak preventer 7 is arranged so as to substantially cover the entire rear opening Q from the lower side, having the SMS side to be an upper side.

The feces stopping member 6 prevents the received feces from transferring to a front side to leak out of the second leak preventer and mixing with urine, and prevents the urine received in the urine receiving part from transferring to a rear side to enter into of the second leak preventer and mixing with the feces.

In the front part of the absorbent article 1, the top sheet 4 is positioned below the peripheral shape retaining members 19 and the outer leg gathers 18, and in a lateral center part thereof, the top sheet 4 is exposed on the surface (see FIG. 16).

In the longitudinal center part of the absorbent article 1, the bridging member 5 is exposed and its right and left ends are bonded to back surfaces of the peripheral shape retaining members 19. The feces stopping member 6 is positioned therebelow, and the second leak preventer 7 to be bonded to the feces stopping member 6 is positioned therebelow (see FIG. 17).

In the rear part of the absorbent article 1, the second leak preventer 7 is exposed, and its right and left ends are bonded to side sheets forming the peripheral shape retaining members 19 and the outer leg gathers 18.

The absorbent article 1 has a structure in which the stretchable material arranged in a lateral center part is sandwiched between two SMS' s. Thus, the bridging member 5 itself is stretchable in the lateral center part. The peripheral shape retaining members 19 each have a structure including the urethane filaments. Thus, the peripheral shape retaining members 19 themselves are entirely stretchable.

Thus, the stretchability of the bridging member 5 itself and the stretchability of the peripheral shape retaining members 19 to which the bridging member 5 is integrally bonded allow the bridging member 5 to maintain a state in contact with a perineal region of the wearer following body motions of the wearer even during motion such as opening and closing of legs of the wearer. In this way, urine and feces can be separated efficiently.

As shown in FIGs. 15 and 19, a front sealing member 13 and a rear sealing member 14 are arranged in front and rear parts of the absorbent article 1 so as to across over the right and left peripheral shape retaining members 19. The front sealing member 13 and the rear sealing member 14 are each formed of PPSB (available from Toray Saehan Inc., mass per unit of 13 g/m², for example) laminated with a PE film and are each bonded to the top sheet 4 on a front end or a rear end.

The front sealing member 13 is bonded to the base part of the first leak preventer 2 and parts of the pair of side members from their front ends to upper front sides to close the front opening of the first leak preventer 2. The rear sealing member 14 is bonded to the base part of the first leak preventer 2 and parts of the pair of side members from their rear ends to upper rear sides to close the rear opening of the first leak preventer 2.

The front sealing member 13 prevents the excreted urine from leaking out from the front opening of the first leak preventer 2, and the rear sealing member 14 prevents the excreted feces from leaking out from the rear opening of the first leak preventer 2.

A waist band 20 is arranged in a vicinity of a rear end of the absorbent article 1. The waist band 20 has a structure in which 15 urethane filaments (available from DuPont-Toray Co., Ltd., 470 dtex, for example) are sandwiched by two SMS's (available from Avgol Ltd., weight per unit area of 13 g/m², for example).

Hook members 15 are provided on right and left ends of the waist band 20 and are removable together with a loop member 16 provided under the first leak preventer 2 in a front side.

As shown in FIG. 21, the absorbent article 1 forms a waist band-type diaper in which the hook members 15 and the loop member 16 are bonded to each other form a waist hole W when the absorbent article 1 is worn.

### <Embodiment 4>

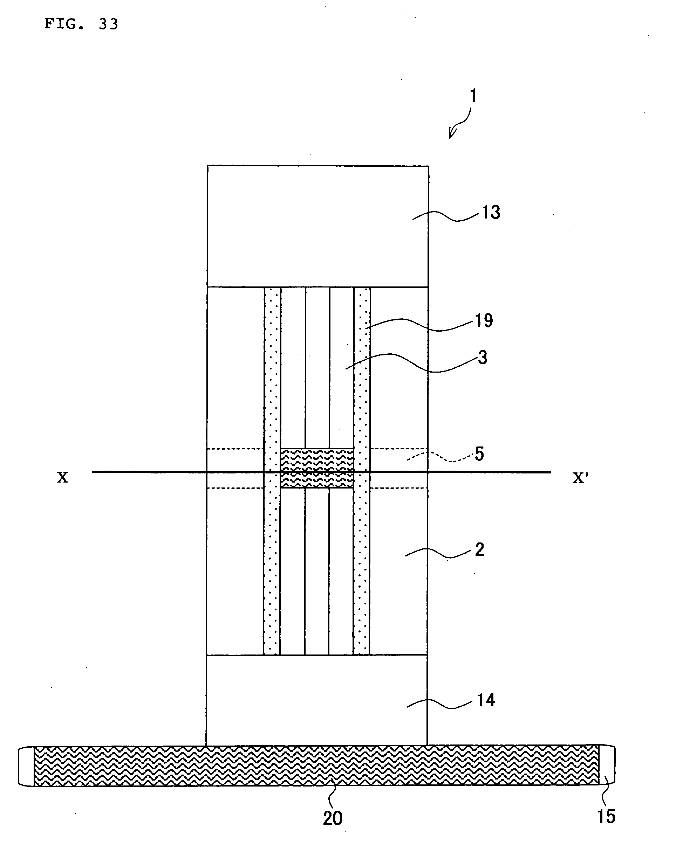

FIGs. 33 to 35 are schematic diagrams showing an absorbent article of the present invention according to another embodiment of a waist band-type diaper.

FIG. 33 is a developed plan view. Note that in FIG. 33, a front side of the absorbent article is positioned on an upper side of the drawing.

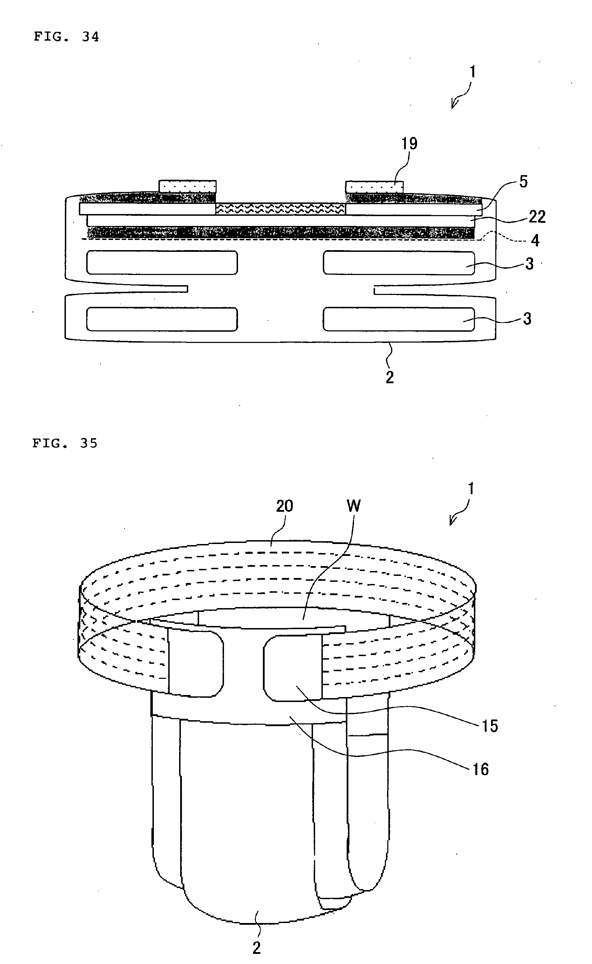

FIG. 34 is a lateral end view of the absorbent article taken along the X-X' line of FIG. 33.

FIG. 35 is aperspective view showing a situation that the absorbent article is worn.

In FIG. 34, a shaded part represents an adhesive.

The absorbent article 1 shown in FIG. 33 basically includes: the first leak preventer 2 in sheet form having a base part; a pair of side members formed of a pair of extended parts of the first leak preventer formed integrally with the base part on right and left sides of the base part of the first leak preventer 2 and a pair of peripheral shape retaining members 19 provided along peripheries of the respective extended parts; the absorber 3 arranged in an inner space formed by the base part of the first leak preventer 2 and the side members, containing a super absorbent polymer, and capable of absorbing a body fluid; and the bridging member 5 bonded to inner surfaces of the pair of side members for bridging the side members; and the urine/feces separating member 22 for dividing the inner space into front and rear parts, capable of exhibiting a function of separating urine and feces, in which the bridging member 5 has stretchability in a lateral center part (shown by chain lines) such that the bridging member 5 is stretchable in a lateral direction.

In the absorbent article 1 shown in FIG. 33, the first leak preventer 2 in sheet form having a base part has the pair of side members extending in a longitudinal direction on right and left sides of the base part being folded inward. On peripheries of the side members of the first leak preventer 2, the peripheral shape retaining members 19 are provided across substantially the entire length.

When the absorbent article of the present invention is worn, front parts of the peripheral shape retaining members 19 are positioned on right and left sides and front side of a urethral opening and upper surfaces of the peripheral shape retaining members are brought into contact with a wearer's body, to thereby fix the absorbent article flexibly, prevent the urethral opening from becoming into direct contact with the absorbent article, or prevent high pressure on the urethral opening even if the urethral opening and the absorbent article are brought into direct contact with each other. In this way, pressure on the urethral opening can be eliminated when the absorbent article is worn. Further, a space formed between the right and left peripheral shape retaining members 19 serves as a passage of urine. Thus, only the urethral opening and its vicinity of the wearer get wet and no other parts get wet on urination.

On the front side of the absorbent article 1, genital organs are held between the right and left peripheral shape retaining members 19, and the front sides of peripheral shape retaining members 19 contact closely to the wearer's body.

On the rear side of the absorbent article 1, the peripheral shape retaining members 19 contact closely to the wearer' s skin along roundness of the buttocks.

The side members of the first leak preventer 2 are formed to be pleated and are divided into two stages at a folded part halfway. In this way, a larger amount of urine can be absorbed, and the absorbent article can be worn for a long period of time.

On lower stage and upper stage of the first leak preventer 2, a absorber 3 in substantially rectangular shape is arranged separately on right and left sides. The absorber 3 is present separately on the right and left sides, and a volume of a center part of the absorber 3 increases when the absorber 3 absorbs urine.

Above the upper stage of the absorber 3 on right and left sides, the top sheet 4 is arranged over a lateral center part. The top sheet 4 may employ the same top sheet as that of Embodiment 1.

In the vicinity of a longitudinal center part, the bridging member 5 is provided. The urine/feces separating member 22 is bonded to a back surface of the bridging member 5. A back side of the urine/feces separating member 22 is bonded to the top sheet 4 through an adhesive. Thus, the inner space is divided into front and rear parts. The front part serves as the urine receiving part, and the rear part serves as the feces receiving part.

As shown in FIG. 33, the front sealing member 13 and the rear sealing member 14 are arranged in front and rear parts respectively of the absorbent article 1, bridging the right and left peripheral shape retaining members 19, similar to Embodiment 3.

As shown in FIG. 35, the absorbent article 1 forms a waist band-type diaper in which the hook members 15 and the loop member 16 are bonded to each other to form a waist hole W when the absorbent article 1 is worn, similar to Embodiment 3.

### <Embodiment 5>

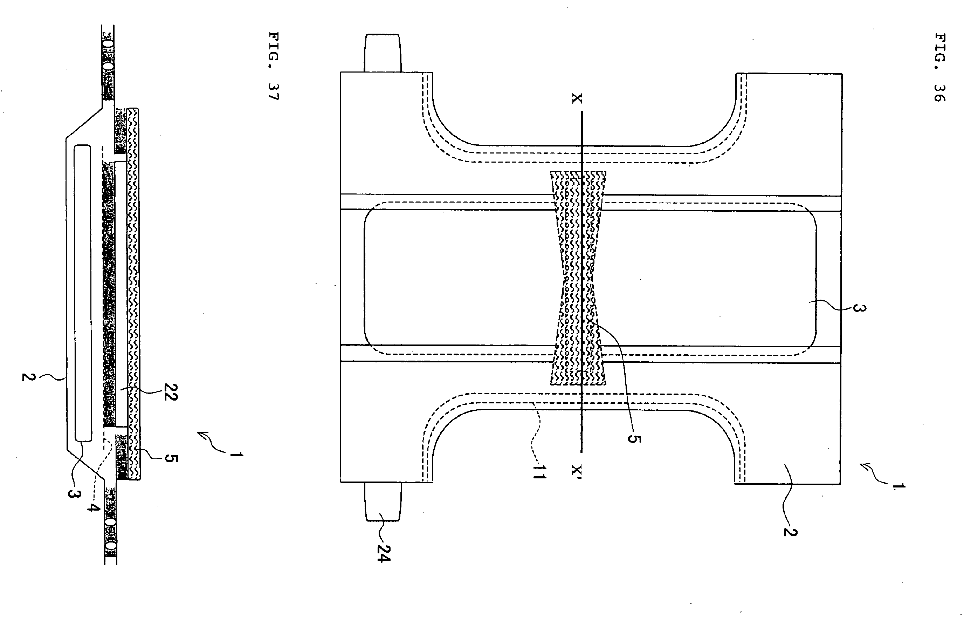

FIGs. 36 to 38 are schematic diagrams showing an absorbent article of the present invention according to another embodiment of a tape-type diaper.

FIG. 36 is a developed plan view. Note that in FIG. 36, a front side of the absorbent article is positioned on an upper side of the drawing.

FIG. 37 is a lateral end view of the absorbent article taken along the X-X' line of FIG. 36.

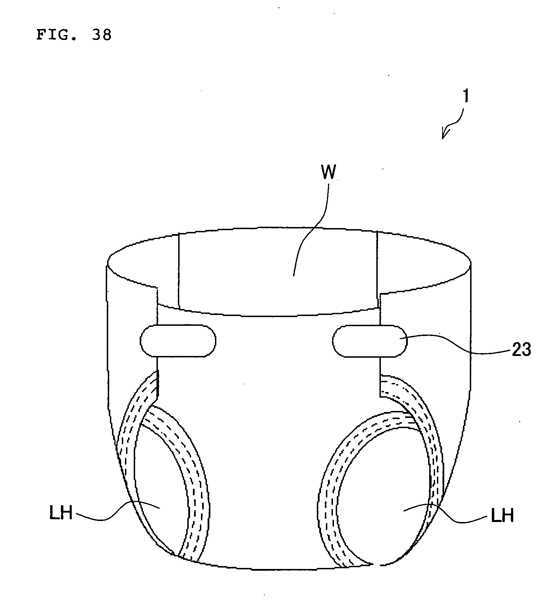

FIG. 38 is a perspective view showing a situation that the absorbent article is worn.

In FIG. 37, a shaded part represents an adhesive.

The absorbent article 1 shown in FIG. 36 basically includes: the first leak preventer 2 in sheet form having a base part; a pair of side members formed of leg gathers 11 provided on right and left ends of the base part of the first leak preventer 2 and extending in a longitudinal direction; the absorber 3 arranged in an inner space formed by the base part of the first leak preventer 2 and the side members, containing a super absorbent polymer, and capable of absorbing a body fluid; and the bridging member 5 bonded to outer surfaces of the pair of side members for bridging the side members; and the urine/feces separating member 22 for dividing the inner space into front and rear parts, capable of exhibiting a function of separating urine and feces, in which the bridging member 5 is entirely stretchable so that the bridging member 5 is stretchable in a lateral direction.

In the absorbent article 1 shown in FIG. 36, the absorber 3 in sheet form is arranged on the first leak preventer 2 in sheet form having a base part and forming leg holes LH on right and left sides, and the top sheet 4 is arranged thereon. The top sheet 4 may employ the same top sheet as that of Embodiment 1.

In the vicinity of a longitudinal center part, the bridging member 5 is provided. The urine/feces separating member 22 is bonded to a back surface of the bridging member 5. A back side of the urine/feces separating member 22 is bonded to the top sheet 4 through an adhesive. Thus, the inner space is divided into front and rear parts. The front part serves as the urine receiving part, and the rear part serves as the feces receiving part.

At right and left ends of the base part of the first leak preventer 2, the leg gathers 11 extend in a longitudinal direction. The leg gathers 11 may employ the same leg gathers as those of Embodiment 2.

As shown in FIG. 38, in the absorbent article 1 worn, the front and rear ends of the first leak preventer 2 are bonded to each other through a bonding tape 23 to form one waist hole W and two leg holes LH.

### <Embodiment 6>

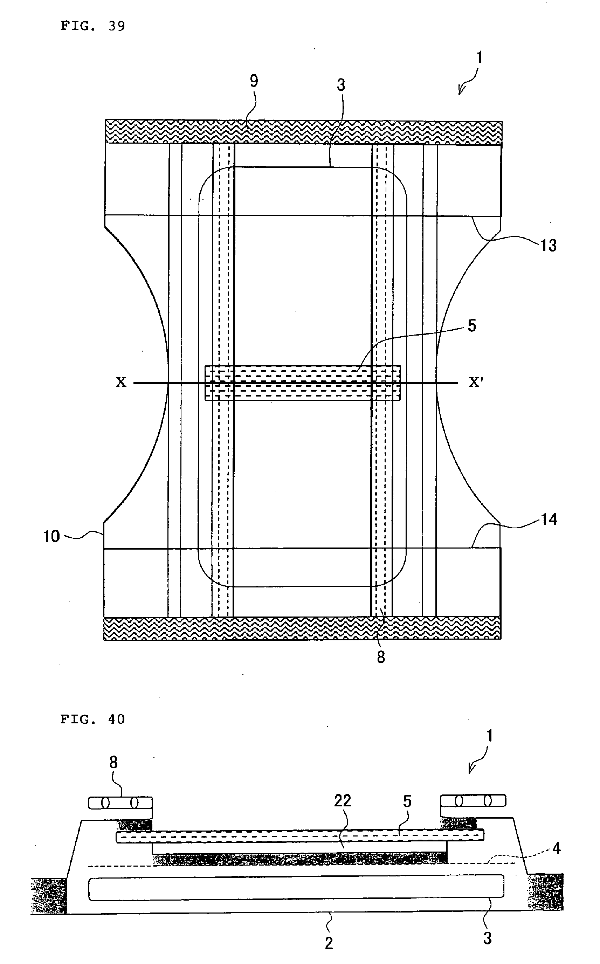

FIGs. 39 to 41 are schematic diagrams showing an absorbent article of the present invention according to another embodiment of a pull-on type diaper.

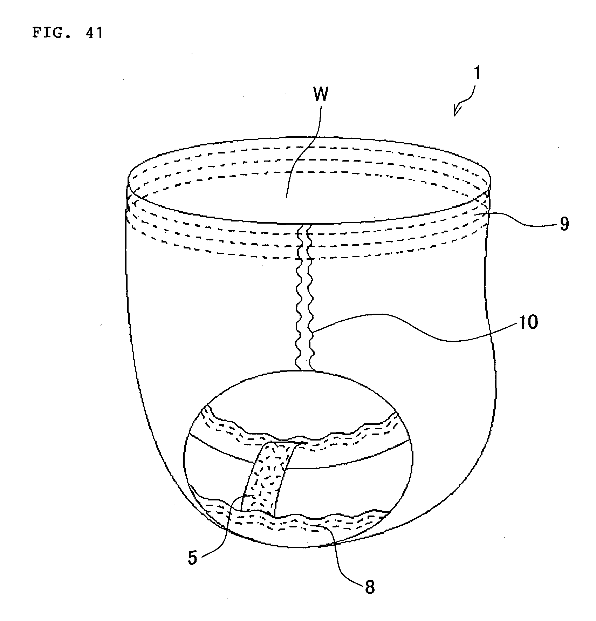

FIG. 39 is a developed plan view. Note that FIG. 39 is a developed view obtained by cutting in a vicinity of the bonding part 10 shown in FIG. 41. In FIG. 39, a front side of the absorbent article is positioned on an upper side of the drawing.

FIG. 40 is a lateral end view of the absorbent article taken along the X-X' line of FIG. 39.

FIG. 41 is aperspectiveview showing a situation that the absorbent article is worn.

In FIG. 40, a shaded part represents an adhesive.

The absorbent article 1 of the present invention basically includes: the first leak preventer 2 in sheet form having a base part; a pair of side members provided on right and left ends of the base part of the first leak preventer 2 and formed of standing leg gathers 8 each including a leg part bonded to the base part and formed of a non-woven fabric and a head part bonded to the leg part and including an elastic body; the absorber 3 arranged in an inner space formed by the base part of the first leak preventer 2 and the standing leg gathers 8, containing a super absorbent polymer, and capable of absorbing a body fluid; and the bridging member 5 bonded to the standing leg gathers 8 as the pair of the side members for bridging; and the urine/feces separating member 22 for dividing the inner space into front and rear parts, capable of exhibiting a function of separating urine and feces, in which the bridging member 5 and the standing leg gathers 8 have stretchability such that the bridging member 5 is stretchable in a lateral direction.

In the absorbent article 1 shown in FIG. 39, the absorber 3 in sheet form is arranged on the first leak preventer 2 in sheet form having a base part and forming leg holes on right and left sides, and the top sheet 4 is arranged thereon. The top sheet 4 may employ the same top sheet as that of Embodiment 1.

In the vicinity of a longitudinal center part, the bridging member 5 is provided. The urine/feces separating member 22 is bonded to a back surface of the bridging member 5. A back side of the urine/feces separating member 22 is bonded to the top sheet 4 through an adhesive. Thus, the inner space is divided into front and rear parts. The front part serves as the urine receiving part, and the rear part serves as the feces receiving part.

The bridging member 5 has a structure in which a stretchable material formed of three urethane filaments (available from DuPont-Toray Co., Ltd., 470 dtex, for example) is arranged in a lateral center part between two SMS's (available from Avgol Ltd. , weight per unit area of 13 g/m², for example).

On right and left sides of the base part of the first leak preventer 2, the standing leg gathers 8 serving as a pair of side members extend in a longitudinal direction. The standing leg gathers 8 may employ the same standing leg gathers as those of Embodiment 1.

As shown in FIG. 39, the front sealing member 13 and the rear sealing member 14 are arranged in front and rear parts of the absorbent article 1 across over the right and left standing leg gathers 8, similar to Embodiment 3.

In the absorbent article 1, the bridgingmember 5 itself is entirely stretchable. Further, the standing leg gathers 8 themselves are entirely stretchable.

Thus, the stretchability of the bridging itself 5 and the stretchability of the standing leg gathers 8 to which the bridging member 5 is bonded maintain the bridging member 5 in contact with a perineal region of the wearer following body motions even during motion of the wearer such as opening and closing of legs of the wearer. In this way, urine and feces can be separated efficiently.

As shown in FIG. 41, in the absorbent article 1, the front body part and the back body part are bonded to each other at the bonding part 10, and the absorbent article 1 forms a pull-on type diaper provided with the waist gather 9 forming the waist hole W.

As described above, the absorbent article of the present invention has been described in detail based on embodiments shown in the drawings. However, the present invention is not limited thereto, and structures of the respective members may be replaced by arbitrary structures which may exhibit similar functions.

The structures of the respective members of embodiments may be combined arbitrarily as another embodiment.

The absorbent article of the present invention may preferably be used as an absorbent article for an adult male, an adult female, or a child.

### [Examples]

Hereinafter, the present invention will be described in detail by referring to examples. However, the present invention is not limited thereto.

### 1. Absorbent article

In Examples, the absorbent articles of the present invention shown in FIGs. 1 to 7 and the absorbent articles shown in FIGs. 15 to 21 were used. A super absorbent sheet prepared by coating SAP-dispersed slurry on a non-woven fabric (MegaThin (trademark), available from Japan Absorbent Technology Institute, weight per unit area of SAP: 180 g/m²) was used as an absorber. The absorber had a length of 350 mm and a width of 160 mm, and was arranged in the absorbent article as being folded its right and left ends inward to form a C-shape.

The super absorbent sheet in the absorbent article had an SAP amount of 10 g, a designed free water-absorption amount of 500 mL, and a designed dehydrated water-absorption amount of 300 mL.

Note that the designed free water-absorption amount was determined in accordance with JIS K7223-1996 "Testing method for water-absorption capacity of super absorbent polymers". The designed dehydrated water-absorption amount was determined by measuring the designed free water-absorption amount and then centrifuging the absorbent article at 1,000 G for 10 min for dehydration.

### 2. Test on Wearability

The absorbent article was worn by each of six infants (three boys and three girls) as wearers each having a healthy urination mechanism and a healthy defecation mechanism, and the wearers each wore the absorbent article in usual days until after defecation when possible.

In this way, the test on wearability was conducted, and studies were conducted when awake or in sleep and posture of the wearer, wearing time length, absorption amounts of urine and feces, presence or absence of defecation, type of feces, state of urine/feces separation, and soil in a vicinity of genital organs and anus.

The test on wearability was conducted four times for each wearer (the test on wearability for the second to fourth times each was conducted with a replaced new absorbent article).

Table 1 and Table 2 show results of the total of 24 tests on wearability of the absorbent articles of the present invention shown in FIGs. 1 to 7. Table 3 and Table 4 show results of the total of 40 tests on wearability of the absorbent articles of the present invention shown in FIGs. 15 to 21.

Table 1 and Table 3 show the main posture, the wearing time lengths (average), the absorption amounts of urine and feces (average), the number of uses, and the number of defecation (number of absorbent articles soiled by defecation) each when the wearer is awake and in sleep.

Table 2 and Table 4 show the number of success and success rate of urine/feces separation, and the soiled state in the vicinity of genital organs and anus by the type of feces and by gender for the cases defecation was seen. Note that the type of feces was classified into normal feces, and soft feces or watery feces depending on an amount of water in the feces. Evaluation on state of urine/feces separation was indicated as success or failure depending on whether or not the feces was separated and received in the feces receiving part.
[Table 1]

**Table 1**

| Awake or in sleep | Main posture | Wear-in g time (h) | Absorption amounts of urine and feces (g) | Number of articles used | Number of articles soiled with feces |
|---|---|---|---|---|---|
| Awake | Standing, Sitting, Walking, Crawling, Lying face down | 5.5 | 205 | 13 | 10 |
| In sleep | Lying face up, Lying sideways | 7.5 | 220 | 11 | 5 |
| - | Average | 6.4 | 211 | - | - |

[Table 2]

**Table 2**

| | Total | Normal feces | | Soft feces/watery feces | |
|---|---|---|---|---|---|
| | Number of success in separation/ Number of uses (separation success rate) | Number of success in separation/ Number of uses (separation success rate) | Soiled state | Number of success in separation/ Number of uses (separation success rate) | Soiled state |
| Total | 12/15 | 8/8 | Substantially | 4/7 | Soiled in vicinity of anus |
| | (80%) | (100%) | not soiled | (57%) | |
| Boys | 7/8 | 5/5 | Substantially | 2/3 | Soiled in vicinity of anus |
| | (88%) | (100%) | not soiled | (67%) | |
| Girls | 5/7 | 3/3 | Substantially | 2/4 | Soiled in vicinity of anus |
| | (71%) | (100%) | not soiled | (50%) | |

[Table 3]

**Table 3**

| Awake or in sleep | Main posture | Wearing time (h) | Absorption amounts of urine and feces (g) | Number of articles used | Number of articles soiled with feces |
|---|---|---|---|---|---|
| Awake | Standing, Sitting, Walking, Crawling, Lying face down | 6.8 | 214 | 23 | 14 |
| In sleep | Lying face up, Lying sideways | 8.1 | 228 | 17 | 4 |
| - | Average | 7.4 | 220 | - | - |

[Table 4]

**Table 4**

| | Total | Normal feces | | Soft feces/watery feces | |
|---|---|---|---|---|---|
| | Number of success in separation/ Number of uses (separation success rate) | Number of success in separation/Nu mber of uses (separation success rate) | Soiled state | Number of success in separation/ Number of uses (separation success rate) | Soiled state |
| Total | 14/18 | 5/5 | Substantially | 9/13 | Soiled in vicinity of anus |
| | (78%) | (100%) | not soiled | (69%) | |
| Boys | 6/8 | 2/2 | Substantially | 4/6 | Soiled in vicinity of anus |
| | (75%) | (100%) | not soiled | (67%) | |
| Girls | 8/10 | 3/3 | Substantially | 5/7 | Soiled in vicinity of anus |
| | (80%) | (100%) | not soiled | (71%) | |

Tables 1 and 2 as well as Tables 3 and 4 reveal that in the case where the absorbent articles of the present invention were used, urine/feces separation succeeded 100% for normal feces and succeeded 57% (in the case of the absorbent articles of the present invention shown in FIGs. 1 to 7) or by 69% (in the case of the absorbent articles of the present invention shown in FIGs. 15 to 21) for soft feces or watery feces which generally spreads over the diaper. Substantially no soil was present for normal feces, and soil in the vicinity of anus alone was present for soft feces or watery feces. No soil was present in a large area.

## Claims

1. An absorbent article including:
a first leak preventer in sheet form having a base part;
a pair of side members extending in a longitudinal direction on right and left sides of the base part of the first leak preventer;
an absorber comprised of at least one layer in an inner space formed by the base part of the first leak preventer and the side members, containing a super absorbent polymer, and capable of absorbing a body fluid; and
a bridging member bonded to the pair of side members for bridging the side members, dividing the inner space into front and rear parts, capable of exhibiting a function of separating urine and feces, in which
the bridging member is formed to be stretchable in a lateral direction.

2. The absorbent article according to claim 1, including a main body bonding member capable of bonding to the first leak preventer or the absorber at a lower end of the bridging member.

3. An absorbent article including:
a first leak preventer in sheet form having a base part;
a pair of side members extending in a longitudinal direction on right and left sides of the base part of the first leak preventer;
an absorber comprised of at least one layer in an inner space formed by the base part of the first leak preventer and the side members, containing a super absorbent polymer, and capable of absorbing a body fluid; and
a bridging member bonded to the pair of side members for bridging the side members; and
a urine/feces separating member dividing the inner space into front and rear parts, capable of exhibiting a function of separating urine and feces, in which
the bridging member is formed to be stretchable in a lateral direction.

4. An absorbent article including:
a first leak preventer in sheet form having a base part;
a pair of side members extending in a longitudinal direction on right and left sides of the base part of the first leak preventer;
an absorber comprised of at least one layer in an inner space formed by the base part of the first leak preventer and the side members, containing a super absorbent polymer, and capable of absorbing a body fluid;
a bridging member bonded to the pair of side members for bridging the side members; and
a second leak preventer in sheet form present above the absorber on a rear side of the first leak preventer, in which
the bridging member is formed to be stretchable in a lateral direction.

5. The absorbent article according to claim 4, including a feces stopping member in an upper surface or in a vicinity of a front end of the second leak preventer.

6. The absorbent article according to claim 4 or 5, including a main body bonding member bonding to the first leak preventer, the absorber, or the second leak preventer at a lower end of the bridging member.

7. The absorbent article according to claim 2, in which the bridging member and the main body bonding member are formed of a same material.

8. The absorbent article according to claim 2, in which the bridging member and/or the main body bonding member is stretchable.

9. An absorbent article according to claim 1, in which the side members are formed of a pair of extended parts of the first leak preventer formed integrally with the base part and a pair of peripheral shape retaining members provided along peripheries of the extended parts.

10. The absorbent article according to claim 1, in which the side members have stretchability.

11. The absorbent article according to claim 10, in which the side members are leg gathers provided on right and left ends of the base part.

12. The absorbent article according to claim 11, in which the leg gathers are standing leg gathers each including a leg part bonded to the base part and formed of a non-woven fabric, and a head part bonded to the leg part and including an elastic body.

13. The absorbent article according to claim 1, in which: the bridging member includes a stretchable area in a center part and non-stretchable areas on right and left sides of the stretchable area; and the bridging member is bonded to the side members in the non-stretchable areas.

14. The absorbent article according to claim 1, in which: the side members are each stretchable; the bridging member is non-stretchable; and the side members are bonded to the bridging member so that the bridging member becomes stretchable in a lateral direction.

15. The absorbent article according to claim 1, in which the side members and the bridging member are integrally formed of a stretchable material.

16. The absorbent article according to claim 15, in which the side members on right and left sides are bonded to each other in a longitudinal center part to form the bridging member.

17. The absorbent article according to claim 15 or 16, in which the side members and the bridging member form a front opening and a rear opening.
